# DEMANDE DE BREVET EUROPEEN

(11) **EP 2 275 100 A2**
(43) Date de publication de la demande: **19.01.2011**
(21) Numéro de dépôt: 10185599.7
(22) Date de dépôt: 18.07.2003
(51) Int. Cl.: A61K 31/155, A61K 31/4245, A61K 31/506, A61P 33/06, A61P 33/00

(54) **Composés à activité antiparasitaire et médicaments les renfermant**

(30) Priorité: 18.07.2002 FR 0209156
(62) Demande divisionnaire de: 03750822.3
(71) Demandeur: Centre National de la Recherche Scientifique (CNRS), 75794 Paris (FR)
(72) Inventeur: Vial, Henri, 34060, MONTPELLIER (FR); Calas, Michèle, 34090, MONTPELLIER (FR); Escale, Roger, 34790, GRABELS (FR); Vidal, Valérie, 34090, MONTPELLIER (FR); Bressolle, Françoise, 34000, MONTPELLIER (FR); Ancelin, Marie-Laure, 34270, ST JEAN DE CUCULLES (FR)
(74) Mandataire: Le Coupanec, Pascale A.M.P.

(57) **Abrégé**

L'invention vise des composés présentant une activité anti-parasitaire, notamment antipaludique, **caractérisés en ce qu'**ils répondent à la formule générale (I)

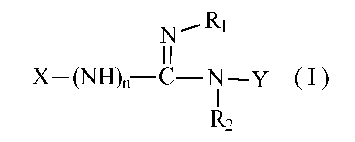

Applications notamment comme composés à activité anti-parasitaire.

## Description

L'invention a pour objet des composés à activité anti-parasitaire, et plus spécialement antipaludique et antibabésiose.

De nombreux travaux sont consacrés à la recherche de médicaments actifs contre les parasites et spécialement contre les *Plasmodium,* en particulier *Plasmodium falciparum,* compte tenu de l'extension des maladies qu'ils provoquent.

Les inventeurs ont constaté, avec certaines catégories de composés chimiques, une activité élevée, s'accompagnant d'une toxicité tolérable et de propriétés de biodisponibilité élevées. Avantageusement, ces composés sont administrables par voie orale.

L'invention a donc pour but de founir de nouveaux composés à activité anti-parasitaire, en particulier antipaludique.

Elle vise également un procédé de synthèse de tels composés.

L'invention vise en outre les médicaments renfermant de tels composés en tant que principes actifs ainsi que leur utilisation et celle de dérivés pour la fabrication de médicaments présentant lesdites activités.

Les composés selon l'invention sont **caractérisés en ce qu**'ils répondent à la formule générale (I) 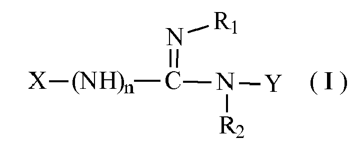 dans laquelle
- soit X représente un groupe de formule (II) 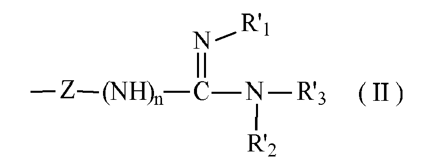
   où Z est un groupe -(CH₂)ₘ, avec m = 8 à 21,
   n= 0 ou 1
   et Y = R₃,
      . R₁ et R'₁, identiques ou différents l'un de l'autre, étant choisis parmi H, alkyl, OH, O-alkyl, O-aryl, O-CO-alkyl, O-CO-aryl, OSO₂-alkyl, OSO₂-aryl, OSO₂-hétérocycle, O-CO-O(ou S ou NH)-alkyl, O-CO-O(ou S ou NH)-aryl, PO(O-alkyl ou O-aryl)₂, CO-O-CH₂-aryl, cycloalkyl,
      . R₂ et R'₂, identiques ou différents l'un de l'autre, étant choisis parmi H, alkyl, CO-O-CH₂-aryl, CO-O-alkyl, cycloalkyl,
      . R₃ et R'₃, identiques ou différents l'un de l'autre, représentant H, alkyl, CO-O-alkyl, CO-O-aryl, COO-CH(R)-O-CO-alkyl, PO(O-alkyl ou O-aryl ou ONa)₂, CO-O-CH(R)-aryl,
      . R étant H ou alkyl, ou bien
      . R₁ et R₂, et/ou R'₁ et R'₂, ou R₂ et R₃ et/ou R'₂ et R'₃, forment ensemble un mono hétérocycle non aromatique avec les atomes d'azote auxquels ils sont respectivement attachés, ou encore,
      . R₂ et R₃ et/ou R'₂ et R'₃ peuvent être le même substituant, doublement lié à l'azote, cyclisé avec, respectivement, R₁ ou R'₁ pour former un hétérocycle, le cas échéant substitué par Rₐ, qui est choisi parmi H, alkyl, alkyl substitué par 1,2 ou 3 atomes d'halogène, aryl, CO-O-alkyl (ou aryl), -CO-OH, -CO-NH₂, -CN, -CO-NH-alkyl (ou aryl), -CO-N-(alkyl)₂, -CO-hétérocyle azoté et/ou oxygéné, NH(H ou alkyl), N(alkyl)₂, hétérocyle azoté et/ou oxygéné, -O-alkyl (ou aryl), -O-CH₂-aryl, CH₂N[H, (H, alkyl), (dialkyl), aryl], -CH₂-hétérocycle azoté et/ou oxygéné, CH₂-CO-OH,
- soit X = R₄ et Y représente un groupe de formule (III) 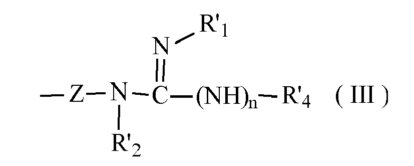
   avec n et Z tels que définis ci-dessus,
   . R₁ et R'₁, identiques ou différents l'un de l'autre, étant choisis parmi H, alkyl, OH, O-alkyl, O-aryl, O-CO-alkyl, O-CO-aryl, OSO₂-alkyl, OSO₂-aryl, OSO₂-hétérocycle, O-CO-O(ou S ou NH)-alkyl, O-CO-O(ou S ou NH)-aryl, PO(O-alkyl ou O-aryl)₂, CO-O-CH_{2.}aryl, cycloalkyl,
   . R₄ et R'₄ représentent H, alkyl ou aryl, ceux-ci pouvant être substitués par OH, O-alkyl, O-aryl, NH (H ou alkyl), hétérocycle azoté et/ou oxygéné et
   . R₂ et R'₂, identiques ou différents l'un de l'autre, étant choisis parmi H, alkyl, CO-O-CH₂-aryl, CO-O-alkyl, cycloalkyl,ou
   . R₁ et R₄ et/ou R'₁ et R'₄ forment ensemble un groupe -(CH₂)ₚ, p étant un entier de 1 à 5, un ou plusieurs atomes d'hydrogène pouvant être remplacés par un alkyl inférieur, et R₂ et R'₂ représentant H, ou bien R₄ et R₂ et/ou R'₄ et R'₂ forment ensemble un groupe -(CH₂)ₚ, 1 ou plusieurs H pouvant être remplacés par un alkyl inférieur, R₁ et R'₁ représentant H, et les sels pharmacologiquement acceptables de ces composés.
      A moins de précisions contraires,
- "aryle" désigne un phényle ou tout cycle ou hétérocycle, ayant un caractère aromatique, comme les cycles pyridine, oxazole, thiazole, le cas échéant substitué, en particulier de chlore, -NO₂, -NH₂, N(H,alkyle) ou (dialkyle);
- "hétérocycle azoté et/ou oxygéné" désigne un cycle à 5 ou 6 sommets comme les cycles pyrrolidine, pipéridine, morpholine, pipérazine, méthylpipérazine ;
- "alkyle" désigne un alkyle en C1-C5, à chaîne droite ou ramifiée, le cas échéant substitué par un ou plusieurs atomes d'halogène, groupe amino -NH₂, N(H, alkyle) ou (dialkyle).

Une famille préférée de dérivés de l'invention, ou famille A, répond à la formule (IV) 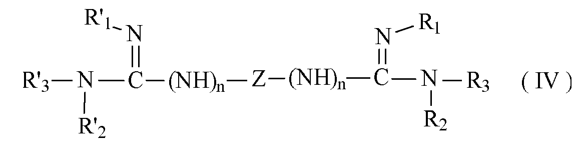 dans laquelle n, Z, R₁,R'₁, R₂,R'₂, R₃ et R'₃ sont tels que définis ci-dessus en rapport avec la formule (II).

Des composés avantageux de cette famille correspondent au cas où n = 0 et répondent à la formule (V) :

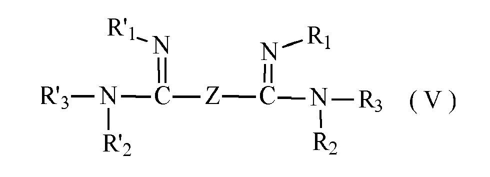

Dans un groupe préféré, ou groupe al, R₁, R₂ et R₃ et/ou R'₁, R'₂ et R'₃ sont indépendants les uns des autres.

Dans un sous-groupe, R₁ et/ou R'₁, et R₂ et/ou R'₂ représentent un atome d'hydrogène, R₃ et/ou R'₃ étant tels que définis ci-dessus, mais différents d'un atome d'hydrogène.

Dans un autre sous-groupe, R₁ et/ou R'₁ sont tels que définis ci-dessus, mais ne représentent pas un atome d'hydrogène, alors que R₃ et/ou R'₃, R₂ et/ou R'₂ représentent un atome d'hydrogène.

Un autre groupe préféré de composés de formule (V) correspond au cas où n = 0 et
- R₁ et R₂, et/ou R'₁ et R'₂, répondent à la formule (VI) ou groupe a2 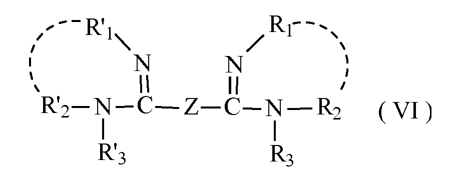 ou
- R₂ et R₃ et/ou R'₂ et R'₃ répondent à la formule (VII) ou groupe a3

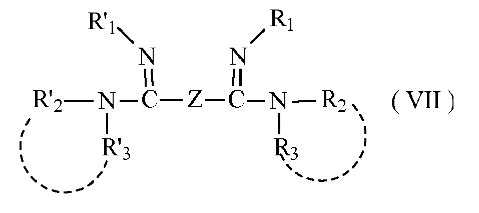

Dans un sous-groupe répondant à la formule (VI), R₁ et R₂ et/ou R'₁ et R'₂ forment ensemble un groupe -O-CO-, O-SO-, O-CS, S-CO ou -S-CS, et R₃ et/ou R'₃ représentent un atome d'hydrogène.

Dans un autre sous-groupe repondant à la formule (VI), R₁ et R₂, et/ou R'₁ et R'₂ représentent un groupe alkylène éventuellement ramifié et R₃ et/ou R'₃ représentent -CO-O-alkyl (ou aryl), -CO-O-CH₂-aryl, CO-O-CH(alkyl)-O-CO-alkyl, PO(O-alkyl ou -aryl)₂, alkyl ou H.

Dans un sous-groupe répondant à la formule (VII),
R₁ et/ou R'₁ représentent un atome d'hydrogène, et
R₂ et R₃, et/ou R'₂ et/ou R'₃ représentent un groupe -(CH₂)ₚ-.

Un autre groupe préféré de la famille A, ou groupe a4, correspond au cas où R₂ et R₃ et/ou R'₂ et R'₃ forment un même substituant et forment ensemble avec R₁ ou respectivement R'₁ un bis-oxadiazole de formule (VIII). 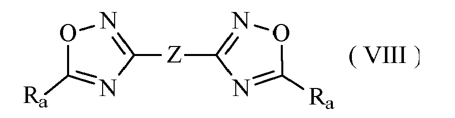 dans laquelle Rₐ est tel que défini ci-dessus.

Dans des composés préférés de ce groupe, l'halogène est avantageusement F ou Cl, l'alkyle est un méthyle ou un éthyle, l'aryle est un phényle.

Une autre famille préférée de l'invention, ou famille B, répond à la formule (IX)

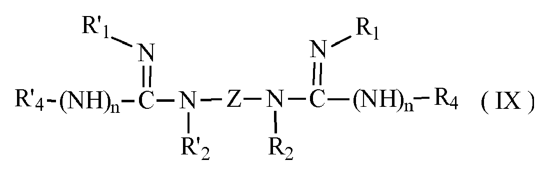

Dans des composés avantageux de cette famille,
Z = -(CH₂)ₘ et n = 0, les composés répondant à la formule (X) :

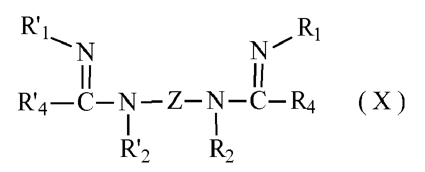

Dans un groupe préféré de la famille B, ou groupe bl, les substituants sont indépendants les uns des autres.

Dans un sous-groupe, R₁ et R₄ et/ou R'₁ et R'₄ sont tels que définis ci-dessus et R₂ représentent un atome d'hydrogène.

Dans un autre sous-groupe, R₁ et R₂ et/ou R'₁ et R'₂ représentent ensemble l'enchaînement oxycarbonyle -OCO- et R₄ et R'₄ sont tels que définis ci-dessus.

Dans encore un autre sous-groupe, R₁ et R'₄ et/ou R'₁ et R'₄ représentent ensemble un un groupe -(CH₂)ₙ- où n est un entier de 3 à 5 et R₂ et R'₂ représentent H.

Dans encore un autre sous-groupe, R₁ et R' représentent H et R₄ et R₂ et/ou R'₄ et R'₂ représentent ensemble un groupe -(CH₂)ₚ- où p est un entier de 3 à 5, un ou plusieurs atomes d'hydrogène pouvant être remplacés par un alkyl inférieur.
Un autre sous-groupe répond à la formule (XI) :

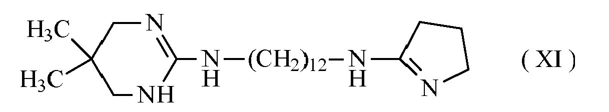

Des composés préférés des familles A et B sont donnés dans les tableaux 1 à 3 ci-après.

**TABLEAU 1**

| | | | | | | |
|---|---|---|---|---|---|---|
| 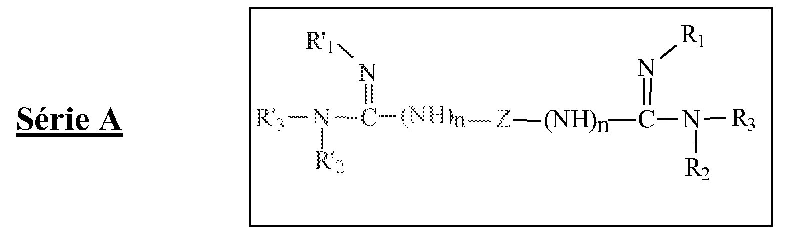 | | | | | | |

| **Composé** | **n** | **Z** | **R₁ (= R'₁)** | **R**₂ (= **R**'₂) | **R**₃ (= **R**'₃) | PM |
|---|---|---|---|---|---|---|
| **1.0, 2HCl** | 0 | -(CH₂)₁₂- | H | H | H | 327 |
| **1.1** | 0 | -(CH₂)₁₂- | H | H | CO₂CH₃ | 370 |
| **1.1, 2HCl** | 0 | -(CH₂)₁₂- | H | H | CO₂CH₃ | 443 |
| **1.2** | 0 | -(CH₂)₁₂- | H | H | CO₂C₂H₅ | 398 |
| **1.3** | 0 | -(CH₂)₁₂- | H | H | CO₂*n*C4H9 | 454 |
| **1.4** | 0 | -(CH₂)₁₂- | H | H | CO₂*iso*C₄H₉ | 454 |
| **1.5** | 0 | -(CH₂)₁₂- | H | H | CO₂CH₂C₆H₅ | 522 |
| **1.6** | 0 | -(CH₂)₁₂- | H | H | CO₂CH₂ pNO₂C₆H₄ | 612 |
| **1.7** | 0 | -(CH₂)₁₂- | H | H | CO₂C₆H₅ | 494 |
| **1.8** | 0 | -(CH₂)₁₂- | H | H | CO₂pFC₆H₄ | 530 |
| **1.8, 2HCl** | 0 | -(CH₂)₁₂- | H | H | CO₂pFC₆H₄ | 603 |
| **1.9** | 0 | -(CH₂)₁₂- | H | H | CO₂pMeOC₆H₄ | 554 |
| **1.10** | 0 | -(CH₂)₁₂- | H | H | CO₂CH₂OCOCH₃ | 486 |
| **1.11** | 0 | -(CH₂)₁₂- | H | H | CO₂CH(CH₃)OCOCH₃ | 514 |
| **1.12** | 0 | -(CH₂)₁₂- | H | H | PO(OC₆H₅)₂ | 718 |
| **1.13** | 0 | -(CH₂)₁₂- | H | H | PO(OC₂H₅)₂ | 526 |
| **1.14** | 0 | -(CH₂)₁₂- | H | H | PO(ONa)₂ | 502 |
| **1.15** | 0 | -(CH₂)₁₂- | OH | H | H | 286 |
| **1.15, 2HCl** | 0 | -(CH₂)₁₂- | OH | H | H | 359 |
| **1.16** | 0 | -(CH₂)₁₂- | OCH₃ | H | H | 314 |
| **1.17** | 0 | -(CH₂)₁₂- | OCOOCH₂CH₃ | H | H | 430 |
| **1.18** | 0 | -(CH₂)₁₂- | OCOOCH₃ | H | H | 402 |
| **1.19** | 0 | -(CH₂)₁₂- | OCOOC₆H₅ | H | H | 526 |
| **1.20** | 0 | -(CH₂)₁₂- | OCOSCH₃ | H | H | 434 |
| **1.21** | 0 | -(CH₂)₁₂- | OCOSCH₂CH₃ | H | H | 462 |
| **1.22** | 0 | -(CH₂)₁₂- | OCOCH₃ | H | H | 370 |
| **1.23** | 0 | -(CH₂)₁₂- | OCOC₆H₅ | H | H | 494 |
| **1.24** | 0 | -(CH₂)₁₂- | OCONHCH₂CH₃ | H | H | 428 |
| **1.25** | 0 | -(CH₂)₁₂- | OCONHC₆H₅ | H | H | 524 |
| **1.26** | 0 | -(CH₂)₁₂- | OPO(OCH₂CH₃)₂ | H | H | 558 |
| **1.27** | 0 | -(CH₂)₁₂- | -O-CO- | | H | 338 |
| **1.28** | 0 | -(CH₂)₁₂- | -O-SO- | | H | 378 |
| **1.29** | 0 | -(CH₂)₁₂- | -O-CS- | | H | 370 |
| **1.30** | 0 | -(CH₂)₁₂- | -S-CO- | | H | 370 |
| **1.31** | 0 | -(CH₂)₁₂- | -S-CS- | | H | 402 |
| **2.0, 2HCl** | 0 | -(CH₂)₁₂- | H | -(CH₂)₄- | | 435 |
| **3.0** | 0 | -(CH₂)₁₂- | H | -(CH₂)₅- | | 390 |
| **4.0, 2HCl** | 0 | -(CH₂)₁₂- | H | CH₃ | CH₃ | 383 |
| **5.0** | 0 | -(CH₂)₁₆- | H | H | H | 382 |
| **6.0** | 0 | -(CH₂)₁₂- | -(CH₂)₂- | | H | 306 |
| **6.1** | 0 | -(CH₂)₁₂- | -(CH₂)₂- | | CO₂CH₃ | 422 |
| **6.2** | 0 | -(CH₂)₁₂- | -(CH₂)₂- | | CO₂C₂H₅ | 450 |
| **6.3** | 0 | -(CH₂)₁₂- | -(CH₂)₂- | | CO₂*n*C₄H₉ | 506 |
| **6.4** | 0 | -(CH₂)₁₂- | -(CH₂)₂- | | CO₂*iso*C₄H₉ | 506 |
| **6.5** | 0 | -(CH₂)₁₂- | -(CH₂)₂- | | CO₂CH₂C₆H₅ | 574 |
| **6.6** | 0 | -(CH₂)₁₂- | -(CH₂)₂- | | CO₂CH₂ pNO₂C₆H₄ | 664 |
| **6.7** | 0 | -(CH₂)₁₂- | -(CH₂)₂- | | CO₂C₆H₅ | 546 |
| **6.8** | 0 | -(CH₂)₁₂- | -(CH₂)₂- | | CO₂pFC₆H₄ | 582 |
| **6.8, 2HCl** | 0 | -(CH₂)₁₂- | -(CH₂)₂- | | CO₂pFC₆H₄ | 655 |
| **6.9** | 0 | -(CH₂)₁₂- | -(CH₂)₂- | | CO₂pMeOC₆H₄ | 606 |
| **6.10** | 0 | -(CH₂)₁₂- | -(CH₂)₂- | | CO₂pNO₂C₆H₄ | 636 |
| **6.11** | 0 | -(CH₂)₁₂- | -(CH₂)₂- | | CO₂CH₂OCOCH₃ | 538 |
| **6.12** | 0 | -(CH₂)₁₂- | -(CH₂)₂- | | CO₂CH(CH₃)OCOCH₃ | 566 |
| **6.13** | 0 | -(CH₂)₁₂- | -(CH₂)₂- | | PO(OC₆H₅)₂ | 770 |
| **6.14** | 0 | -(CH₂)₁₂- | -(CH₂)₂- | | PO(OC₂H₅)₂ | 578 |
| **7.0, 2HCl** | 0 | -(CH₂)₁₂- | -(CH₂)₂- | | CH₃ | 407 |
| **8.0, 2HCl** | 0 | -(CH₂)₁₂- | -(CH₂)₃- | | H | 407 |
| **9.0, 2HCl** | 0 | -(CH₂)₁₂- | -CH(CH₃) | | H | 407 |
| **10.0, 2HCl** | 0 | -(CH₂)₁₂- | -C(CH₃)₂-CH₂- | | H | 435 |
| **11.0, 2HCl** | 0 | -(CH₂)₁₂- | 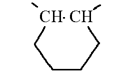 | | H | 487 |
| **12.0, 2HBr** | 1 | -(CH₂)₁₂- | H | H | H | 446 |
| **12.1** | 1 | -(CH₂)₁₂- | CO₂CH₂C₆H₅ | CO₂CH₂C₆H₅ | H | 820 |
| **12.2** | 1 | -(CH₂)₁₂- | CO₂C(CH₃)₃ | CO₂C(CH₃)₃ | H | 684 |
| **13.0, 2HI** | 1 | -(CH₂)₁₂- | H | CH₃ | CH₃ | 370 |
| **14.0, 2HCl** | 1 | -(CH₂)₁₂- | C₆H₁₁ | C₆H₁₁ | H | 685 |
| **15.0, 2HBr** | 1 | -(CH₂)₁₂- | -CH₂-CH₂- | | H | 500 |
| **16.0, 2HI** | 1 | -(CH₂)₁₂- | -CH₂-CH₂-CH₂- | | H | 364 |
| **17.0, 2HI** | 1 | -(CH₂)₁₂- | -CH₂-C(CH₃)₂-CH₂- | | H | 676 |

**TABLEAU 2**

| **Composé** | **Ra** | PM |
|---|---|---|
| **18.0** | H | 306 |
| **18.1** | CH₃ | 334 |
| **18.2** | CHF₂ | 406 |
| **18.3** | CHCl₂ | 472 |
| **18.4** | CF₃ | 442 |
| **18.5** | CCl₃ | 541 |
| **18.6** | C₆H₅ | 458 |
| **18.7** | CO₂C₂H₅ | 450 |
| **18.8** | CO₂H | 394 |
| **18.9** | CONH₂ | 392 |
| **18.10** | CN | 356 |
| **18.11** | CONHCH₃ | 420 |
| **18.12** | CON(CH₃)₂ | 448 |
| **18.13** | CONC₄H₈ | 500 |
| **18.14** | CONC₅H₁₀ | 528 |
| **18.15** | CONC₄H₈O | 532 |
| **18.16** | CONC₄H₈NH | 530 |
| **18.17** | CONC₄H₈NCH₃ | 558 |
| **18.18** | NH₂ | 336 |
| **18.19** | NHCH₃ | 364 |
| **18.20** | N(CH₃)₂ | 392 |
| **18.21** | NC₄H₈ | 444 |
| **18.22** | NC₅H₁₀ | 472 |
| **18.23** | NC₄H₈O | 476 |
| **18.24** | NC₄H₈NH | 474 |
| **18.25** | NC₄H₈NCH₃ | 502 |
| **18.26** | OCH₃ | 366 |
| **18.27** | OC₂H₅ | 394 |
| **18.28** | OCH₂C₆H₅ | 518 |
| **18.29** | CH₂NH₂ | 364 |
| **18.30** | CH₂NHCH₃ | 392 |
| **18.31** | CH₂N(CH₃)₂ | 420 |
| **18.32** | CH₂NC₄H₈ | 472 |
| **18.33** | CH₂NC₅H₁₀ | 500 |
| **18.34** | CH₂NC₄H₈O | 504 |
| **18.35** | CH₂NC₄H₈NH | 502 |
| **18.36** | CH₂NC₄H₈NCH₃ | 530 |
| **18.37** | CH₂NHCOCH₃ | 448 |
| **18.38** | CH₂NHCOOCH₃ | 480 |
| **18.39** | CH₂NHCOOC₆H₅ | 604 |
| **18.40** | CH₂COOH | 422 |

**TABLEAU 3**

| | | | | | | |
|---|---|---|---|---|---|---|
| 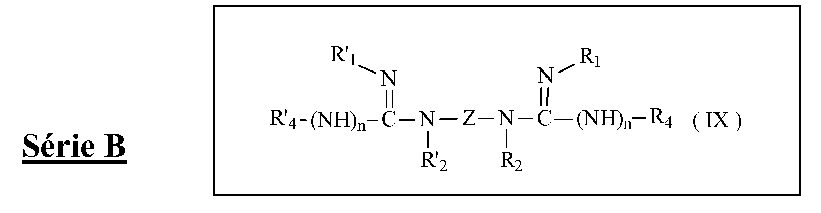 | | | | | | |

| **Composé** | **n** | **Z** | **R**₁ **(= R**'₁) | **R**₄ (= **R**'₄) | **R₂ (= R**'**₂**) | PM |
|---|---|---|---|---|---|---|
| **20.0** | 0 | -(CH₂)₁₂- | H | CH₃ | H | 282 |
| **20.1** | 0 | -(CH₂)₁₂- | OH | CH₃ | H | 298 |
| **20.2** | 0 | -(CH₂)₁₂- | OCH₃ | CH₃ | H | 312 |
| **20.3** | 0 | -(CH₂)₁₂- | OCOOCH₂CH₃ | CH₃ | H | 370 |
| **20.4** | 0 | -(CH₂)₁₂- | OCOOCH₃ | CH₃ | H | 356 |
| **20.5** | 0 | -(CH₂)₁₂- | OCOOC₆H₅ | CH₃ | H | 418 |
| **20.6** | 0 | -(CH₂)₁₂- | OCOSCH₃ | CH₃ | H | 372 |
| **20.7** | 0 | -(CH₂)₁₂- | OCOSCH₂CH₃ | CH₃ | H | 386 |
| **20.8** | 0 | -(CH₂)₁₂- | OCOCH₃ | CH₃ | H | 340 |
| **20.9** | 0 | -(CH₂)₁₂- | OCOC₆H₅ | CH₃ | H | 402 |
| **20.10** | 0 | -(CH₂)₁₂- | OCONHCH₂CH₃ | CH₃ | H | 369 |
| **20.11** | 0 | -(CH₂)₁₂- | OCONHC₆H₅ | CH₃ | H | 417 |
| **20.12** | 0 | -(CH₂)₁₂- | OSO₂CH₃ | CH₃ | H | 470 |
| **20.13** | 0 | -(CH₂)₁₂- | OPO(OC₂H₅) | CH₃ | H | 586 |
| **20.14** | 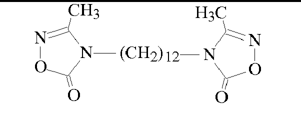 | | | | | 366 |
| **21.0** | 0 | -(CH₂)₁₂- | -(CH₂)₃- | | H | 334 |
| **22.0, 2HCl** | 0 | -(CH₂)₁₂- | -(CH₂)₄- | | H | 435 |
| **23.0** | 0 | -(CH₂)₁₂- | -(CH₂)₅- | | H | 390 |
| **24.0** | 0 | -(CH₂)₁₂- | H | -(CH₂)₃- | | 334 |
| **25.0, 2TFA** | 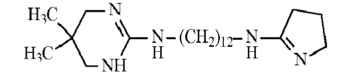 | | | | | 605 |
| **26.0** | 0 | -(CH₂)₁₂- | H | -CH₂OH | H | 298 |

Les composés selon l'invention se présentent le cas échéant sous forme de sels. On citera à titre d'exemples les chlorhydrates, les citrates, les tartrates, les maléates, les lactates, les acétates et trifluoroacétates.

Conformément à l'invention les carbamates et les dérivés N-phosphorylés de formule générale (V) et (VI) définis ci-dessus peuvent être obtenus par un procédé **caractérisé en ce qu**'il comprend la réaction en milieu diphasique des composés bisamidines de formule générale (V) et (VI) dans lesquels R₃ et R'₃ = H avec un dérivé Cl-R₃ (ou R'₃) où R₃ et R'₃ sont tels que définis ci-dessus et différents de H comme illustré dans les exemples.

Les dérivés d'amidoxime de formule générale (V) et (X) définis ci-dessus peuvent être obtenus par un procédé **caractérisé en ce qu**'il comprend la réaction en milieu basique des bisamidoximes de formules générale (V) et (X) dans lesquels R₁ et R'₁ = OH et du réactif approprié comme illustré dans les exemples.

De manière avantageuse des composés de formule générale (VI) groupe a2 et (VIII) groupe a4 définis ci-dessus peuvent être obtenus par cyclisation intramoléculaire d'amidoxime ou de dérivés d'amidoxime précédemment définis de formule générale (V) groupe a1 en présence du réactif approprié comme illustré dans les exemples.

L'étude de l'activité des produits de l'invention vis-à-vis de parasites, et notamment de *Plasmodium,* a montré qu'ils présentent une forte activité *in vitro.*

Ainsi, les valeurs de CI₅₀ (concentration inhibant 50% du parasite) sont de l'ordre du nM au µM vis-à-vis de *P. falciparum.*

L'invention vise donc la mise à profit des propriétés des composés pour l'élaboration de compositions pharmaceutiques.

Les compositions pharmaceutiques de l'invention sont **caractérisées en ce qu**'elles renferment une quantité efficace d'au moins un composé tel que défini ci-dessus, en association avec un véhicule pharmaceutique inerte.

L'invention vise également l'utilisation d'au moins l'un desdits composés pour fabriquer des médicaments pour le traitement de maladies infectieuses, en particulier du paludisme.

Ces compositions renferment le cas échéant des principes actifs d'autres médicaments. On citera notamment leur association avec d'autres antipaludiques (tels que les agents lysosomotropes, l'atovaquone, les antifoliques ou antifoliniques, ou l'artémisinine ou l'un de ses dérivés) pour raison de synergie pharmacologique ou d'évitement de résistance.

On les utilisera également avec avantage en association avec des composés facilitant leur assimilation.

Les compositions pharmaceutiques de l'invention sont administrables sous différentes formes, plus spécialement par voie orale ou injectable ou encore par voie rectale.

Pour l'administration par voie orale, on a recours en particulier à des comprimés, pilules, tablettes, gélules, gouttes.

D'autres formes d'administration comprennent des solutions injectables par voie intraveineuse, sous-cutanée ou intra-musculaire, élaborées à partir de solutions stériles ou stérilisables. Il peut s'agir également de suspensions ou d'émulsions.

On peut également utiliser des suppositoires pour d'autres formes d'administration.

Les compositions de l'invention sont particulièrement appropriées pour le traitement des maladies infectieuses chez l'homme et l'animal, en particulier du paludisme ou des babésioses.

A titre indicatif, la posologie utilisable chez l'homme correspond aux doses suivantes : ainsi on administre par exemple au patient de 1 à 90 mg/kg en une ou plusieurs prises.

L'invention vise encore les réactifs biologiques renfermant comme principes actifs, les composés définis plus haut.

Ces réactifs peuvent être utilisés comme références ou étalons dans des études d'éventuelles activités anti-parasitaires.

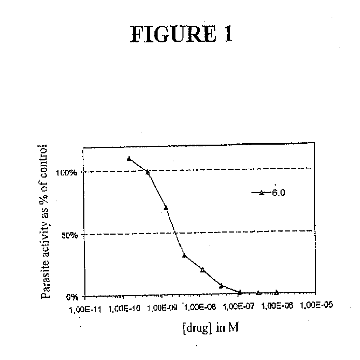

D'autres caractéristiques et avantages de l'invention apparaîtront dans les exemples qui suivent relatifs à la synthèse des composés et à l'étude de leur activité anti-parasitaire. Dans ces exemples, il sera fait référence à la figure 1, qui représente l'activité antipaludique du composé 6.0 en fonction de la concentration en drogue, selon le test de Desjardins, (Desjardins R.E. et al, Antimicrob. Agents Chemother. 1979, 16, 710-718).

### Intermédiaires de synthèse

### 1,12-dicyanododécane:

Une suspension sous agitation vive de 3.3 g (67.10 mmol) de cyanure de sodium dans 30 ml de diméthylsulfoxyde est chauffée entre 90 et 95°C jusqu'à dissolution complète. A la solution obtenue et refroidie à température inférieur à 50°C, sont ajoutés en très petites portions et lentement 10 g (30.49 mmol) de 1,12-dibromododécane. Après 2 H d'agitation à température ambiante la suspension obtenue, est dissoute par addition de 90 ml de dichlorométhane et lavée plusieurs fois par une solution aqueuse saturée de chlorure de sodium. Après quoi, la phase organique séchée sur sulfate de sodium, est évaporée sous pression réduite. Le résidu huileux froid repris dans de l'éther puis évaporé à sec, se cristallise et donne 6.44 g (96 %) de cristaux blancs.
Point de fusion : < 40° C
RMN ¹H (CDCl₃, 100 MHz), δ (ppm): 1.24 (s, 16H); 1.60 (m, 4H); 2.31 (t, 4H) FT-IR, ν (cm⁻¹) : 2246 (CN)

### Dichlorhydrate de diéthyltétradécanediimidoate:

Dans une solution refroidie à 0°C, de 20 ml d'éthanol anhydre, 60 ml d'éther anhydre et de 5 g (22.73 mmol) de 1,12-dicyanododécane, faire barboter pendant 2H de l'acide chlorhydrique gaz puis, laisser le mélange réactionnel sous agitation toute une nuit. Après quoi, les solvants sont évaporés sous pression réduite. Le résidu solide obtenu est cristallisé et lavé plusieurs fois à l'éther puis séché au dessiccateur. On obtient 7.7 g (82%) d'une poudre blanche.
Point de fusion: 118-120°C
RMN ¹H (DMSO-d₆, 100 MHz), δ (ppm): 1.26 (s, 16H); 1.35 (t, 6H); 1.60 (m, 4H); 2.63 (t, 4H); 4.55 (q, 4H); 11.16 et 12.08 (2s, 4H)
FT-IR, ν (cm⁻¹) : 1097 (C-O); 1651.50 (C=N); 3032 et 3118 (NH₂,Cl)

### N,N'-di-benzyloxycarbonyl-S-méthylisothiourée:

A une solution de 1 g (3.60 mmol) de sulfate de S-méthylisothiourée dans 40 ml d'un mélange diphasique dichlorométhane/solution aqueuse saturée de bicarbonate de sodium(1:1), sous agitation vive, sont additionnés goutte à goutte 2 ml (14.4 mol) de chloroformiate de benzyle. L'agitation est maintenue à température ambiante pendant 25 H. Après quoi, la phase aqueuse est extraite 3 fois avec du dichlorométhane. Les phases organiques réunies, sont ensuite lavées avec de l'eau puis séchées sur sulfate de sodium et évaporées sous pression réduite. Le résidu obtenu est purifié par chromatographie sur colonne de silice (DCM) pour donner 1.06 g (82%) de produit sous forme d'une huile jaune.
RMN ¹H (CDCl₃, 100 MHz), δ (ppm): 2.46 (s, 3H); 5.24 (s, 4H); 7.42 (s, 10H); 11.91 (s, 1H)
FT-IR, ν (cm⁻¹) : 1022 et 1173 (C-O); 1647 (C=N); 1751 (NCO); 3169 (NHCO)

### N,N'-di-tert-butyloxycarbonyl-S-méthylisothiourée:

Une solution diphasique (100 ml) de DCM/NaHCO₃ aqueux saturé contenant 5.81 g (26.60 mmol) de di-tert-butylcarbonate et de 2.53 g (18.20 mmol) d'hémisulfate de S-méthylisothiourée est laissée sous agitation vive durant 48 heures. Après quoi, la phase aqueuse séparée, est extraite avec 2 X 100 ml de DCM. Les phases organiques réunies sont ensuite lavées avec 2 X 200 ml d'eau puis évaporées sous pression réduite. Le résidu est par suite repris avec 100 ml du mélange diphasique DCM/NaHCO₃ aqueux saturé auquel on ajoute 0.55 g (3.85 mmol) d'hémisulfate de S-méthylisothiourée. Le mélange réactionnel est encore laissé sous agitation vive durant 72 heures. Les phases organiques réunies après le même traitement que précédemment, sont séchées sur sulfate de sodium et évaporées sous pression réduite. Le résidu est finalement purifié sur silice (Hexane/CHCl₃5% puis CHCl₃) pour conduire à 3.46 g (90%) de produit sous forme de poudre blanche.
Point de fusion : 123- 124 °C
RMN ¹H (CDCl₃, 100 MHz), δ (ppm): 1.49 (s, 18H); 2.38 (s, 3H); 11.60 (s, 1H)
FT-IR, ν (cm⁻¹) : 1043 et 1252 (C-O); 1667 (C=N); 1765 (NCO); 3337 (NHCO)

### O-chlorométhyl-S-éthyl carbonothioate:

A une solution sous agitation et refroidie entre 0 et 5°C de 44 ml (500 mmol) de chlorométhyl chloroformiate dans 900 ml d'éther, est additionnée goutte à goutte durant 2 heures, une solution de 37 ml (500 mmol) d'éthanethiol et 69.3 ml (500 mmol) de triéthylamine dans 200 ml d'éther. Après quoi, les condition opératoires sont maintenues pendant 30 minutes. Le mélange réactionnel est ensuite laissé sous agitation durant 16 heures à température ambiante puis le précipité formé est filtré et lavé avec de l'éther. Les phases éthérées réunies sont évaporées et le résidu purifié par distillation pour donner 57 g (73%) de produit sous forme de liquide.
Point d'ébullition : 99-100°C /.18 mbar
RMN ¹H (CDCl₃, 100 MHz), δ (ppm):); 1.30 (t, 3H); 2.89 (q, 2H); 5.73 (s, 2H).
FT-IR, ν (cm⁻¹) : 1719 (S-CO-O).

### S-éthyl-O-iodométhyl carbonothioate:

A une solution sous agitation de 106.8 g (712 mmol) d'iodure de sodium et 3 g (35.6 mmol) de bicarbonate de sodium dans 450 ml d'acétone, sont ajoutés directement 55 g (356 mmol) de O-chlorométhyl-S-éthyl carbonothioate. Le mélange réactionnel est ensuite laissé sous agitation et à 40°C durant 4 heures. Le précipité formé est filtré et lavé avec de l'acétone et de l'éther. La phase organique est évaporée et le résidu est partagé entre 1100 ml d'hexane refroidi à 0°C et 500 ml d'eau froide. La phase organique séparée, est ensuite lavée successivement avec 200 ml de bicarbonate de sodium aqueux à 5%, 200 ml de thiosulfate de sodium aqueux à 1% (jusqu'à décoloration de la solution) et 2 X 200 ml d'eau. Après séchage de la phase hexanique sur sulfate de sodium et évaporation sous pression réduite, 81 g (92%) de produit sont obtenus sous forme de liquide jaunâtre sans purification.
RMN ¹H (CDCl₃, 100 MHz), δ (ppm):); 1.30 (t, 3H); 2.89 (q, 2H,); 5.96 (s, 2H).
FT-IR, ν (cm⁻¹) : 1715 (S-C-O).

### O-acétyloxyméthyl-S-éthyl carbonothioate:

A une solution sous agitation et refroidie à -20°C de 26.7 g (325.2 mmol) d'acétate de sodium dans 420 ml de diméthylformamide anhydre, sont additionnés goutte à goutte durant 2 heures 80 g (325.2 mmol) de S-éthyl-O-iodométhyl carbonothioate. Le mélange réactionnel est ensuite laissé sous agitation durant 16 heures à température ambiante puis le précipité formé est filtré et lavé avec 20 ml de diméthylformamide et 40 ml d'éther.

A la phase organique dans une ampoule à décanter, est ajouté 850 ml d'éther et 350 ml d'eau froide. La phase aqueuse est isolée et extraite avec 350 ml d'eau. Les phases éthérées réunies sont ensuite lavées successivement avec 220 ml de bicarbonate de sodium aqueux à 5%, 220 ml d'eau, 2 X 220 ml d'acide chlorhydrique 0.01 N et 220 ml d'eau. Après séchage de la phase organique sur sulfate de sodium et évaporation, le résidu est purifié par distillation pour donner 35 g (60%) de produit sous forme de liquide jaunâtre.
Point d'ébullition : 82-83°C /.0.25 mbar
RMN ¹H (CDCl₃, 100 MHz), δ (ppm): 1.30 (t, 3H); 2.09 (s, 3H); 2.87 (q, 2H); 5.76 (s, 2H).
FT-IR, ν (cm⁻¹) : 1717 (S-CO-O); 1767 (CO-O).

### acétyloxyméthyl chloroformiate:

A la solution de 33 g (185.4 mmol) de O-acethyloxyméthyl-S-éthyl carbonothioate sous agitation et refroidie entre 0-5°C, sont ajoutés 14.90 ml (185.4 mmol) de chlorure de sulfuryle. Les conditions opératoires sont maintenues pendant 15 minutes puis le mélange réactionnel est laissé sous agitation durant 45 minutes à température ambiante. La solution de chlorure de S-éthyle formée est évaporée à température ambiante puis à 20 mbar toute une nuit. Le résidu obtenu est ensuite purifié par distillation pour donner 15.50 g (56%) de produit sous forme de liquide orange.
Point d'ébullition : 75-76°C /.17 mbar
RMN ¹H (CDCl₃, 100 MHz), δ (ppm): 2.12 (s, 3H); 5.76 (s, 2H).
FT-IR, ν (cm⁻¹) : 1724 (CO-O); 1773 (Cl-CO).

### 3,4-dihydro-5-méthoxy-2H-pyrrole:

A la solution de 20,03 g (0,235 mol) de pyrrolidine-2-one dans 85 ml de benzène, chauffée à 70°C sont additionnés goutte à goutte 23 ml (1 équivalent) de diméthylsulfate. Ce mélange réactionnel est chauffé à reflux pendant 3 h.. Après refroidissement à température ambiante, 19 ml d'une solution de soude 15N sont ajoutés au mélange réactionnel. Celui-ci est verser dans une ampoule à décanter, et la phase aqueuse est extraite 3 fois avec du benzène. Les phases organiques réunies sont séchées sur sulfate de sodium et évaporées sous pression réduite. Le résidu est distillé sous vide pour donner le produit, liquide incolore, avec un rendement de 58%.
Point d'ébullition : 37°C sous 10⁻² mbar.
RMN ¹H (CDCl₃, 100 MHz), δ (ppm) : 1,97 (m, 2H) ; 2,41 (t, 2H) ; 3,61 (t, 2H) ; 3,75 (s, 3H)

### Iodure de 2-méthylsulfanyl-3,4,5,6-tétrahydropyrimidinium

Le mélange réactionnel composé d'un équivalent de 3,4,5,6-tétrahydro (*1H*) pyrimidine- 2-thione et 1,2 équivalents de iodométhane dans du méthanol (10 ml/g de thio-urée) est chauffé à reflux pendant 5 heures. Après refroidissement, le méthanol est évaporé sous pression réduite pour donner quantitativement le produit. Le précipité peut ensuite être lavé dans de l'acétone ou de l'éther de pétrole.
Point de fusion: 146-148°C
RMN ¹H (DMSO-d₆, 100 MHz) : 1,94 (m, 2H) ; 2,66 (s, 3H) ; 3,44 (t, 4H) ; 9,53 (s, 2H) ppm.

### 5,5-Diméthyl-3,4,5,6-tétrahydro(1H)pyrimidine-2-thione:

Dans un ballon de 250 ml, 10 ml (167, 2 mmol ; 2 éq.) de disulfure de carbone sont additionnés à la solution de 10 ml (83,6 mmol) de 2,2-diméthyl-1,3-diaminopropane dans 50 ml d'éthanol absolu. Puis, 16,02 g (83,6 mmol) d'EDC sont ajoutés au mélange réactionnel, qui est agité 3 heures à température ambiante. L'éthanol est évaporé, le résidu est repris avec de l'eau et extrait au dichlorométhane. Après séchage sur Na₂SO₄ et évaporation de la phase organique on obtient 11,2 g (93%) de produit (poudre blanche), utilisée sans purification supplémentaire.
Point de fusion : 225-228°C
RMN ¹H (CDCl₃, 100 MHz) : 0,91 (s, 6H); 2,88 (d, 4H); 7,46 (s, 2H) ppm.

### Iodhydrate de 5,5-diméthyl-2-méthylsulfanyl-3,4,5,6-tétrahydropyrimidinium :

Le mélange réactionnel composé d'un équivalent de 5,5-diméthyl-3,4,5,6-tétrahydro(*1H*)pyrimidine-2-thione et 1,2 équivalents de iodométhane dans du méthanol (10 ml/g de thio-urée) est chauffé à reflux pendant 5 heures. Après refroidissement, le méthanol est évaporé sous pression réduite pour donner quantitativement le produit. Le précipité peut ensuite être lavé dans de l'acétone ou de l'éther de pétrole.
.RMN ¹H (CDCl₃, 100 MHz): 0,95 (s, 6H); 2,62 (s, 3H); 3,11 (s, 4H), 8,76 (s, 2H) ppm.

### Chlorhydrate de 1-(N-tert-butyloxycarbonylamino)dodécane 12-ammonium:

Une solution de 4,02 g (18,4 mmol)de di-*tert*-butyldicarbonate dans 60 ml de dioxane est additionnée lentement, goutte à goutte (pendant environ deux heures), à une solution de 15,01 g (75 mmol, 4,1 éq.) de 1,12-dodécanediamine dans un mélange dioxane/eau (150/70ml). Le mélange réactionnel est agité à température ambiante pendant 24 h, l'excès de diamine est filtré et les solvants sont évaporés sous pression réduite. Le résidu est repris avec une solution HCl *1N* et du dichlorométhane. Le précipité formé dans la phase aqueuse est filtré pour donner le dérivé mono-Boc et un peu de diamine, tous deux sous forme de sel de chlorhydrate. Ce solide est recristallisé avec de l'éthanol et de l'éther pour donner 4,56 g (74% de rendement) de sel de diamine mono-protégée, sous forme d'une poudre blanche.
Point de fusion: 153-155°C.
RMN ¹H (CD₃OD, 100 MHz), δ (ppm) : 1,28 (m, 20 H) ; 1,38 (s, 9H) ; 2,91 (m, 4H) SM-FAB+ : [M+H]⁺ : 301

### Iodhydrate de [1-[N-(5, 5-diméthyl-3,4,5,6-tétrahydro pyrimidin-2-yl)amino]-12-(N'-tert-butyloxycarbonylamino)] dodécane:

A la suspension de 1,02 g (3 mmol) de chlorure de 12-(*N*-tert-butyloxycarbonylamino)dodécane-1-ammonium dans 15 ml d'acétonitrile sont ajoutés 0,85 g (3 mmol) d'iodure 5,5-diméthyl-2-méthylsulfanyl-3,4,5,6-tétrahydropyrimidinium et 0,85 ml (2 éq.) de triéthylamine. Ce mélange réactionnel est chauffé à reflux pendant 24h. Après refroidissement à température ambiante, l'amine qui n'a pas réagit est filtrée. Le filtrat est évaporé sous pression réduite et chromatographié sur colonne de silice (éluant : CH₂Cl₂/CH₃OH/NH₄OH 80 :16 :4) pour donner 1,36 g (85%) de produit.
RMN ¹H (CD₃OD, 100 MHz), δ (ppm) : 1,07 (s, 6H) ; 1,37 (m, 29H) ; 3,05 (m, 8H).

### Ditrifluoroacétate de 1-[N-(5,5-diméthyl-3,4,5,6-tétrahydropyrimidin-2-yl)amino]dodécane-12-ammonium:

1,06 g (2 mmol) d' iodhydrate de [1-[*N*-(5,5-diméthyl-3,4,5,6-tétrahydropyrimidin-2-yl)amino]-12-(*N*'-*tert*-butyloxycarbonylamino)]dodécane sont solubilisés dans 15 ml d'une solution TFA/CH₂Cl₂ (3/1). Cette solution est agitée 3 heures à température ambiante ; l'excès d'acide trifluoroacétique sont évaporés sous pression réduite pour donner quantitativement 0,98 g de produit.
RMN ¹H (DMSO-d₆, 100 MHz), δ (ppm) : 1,0 (s, 6H) ; 1,3 (m, 20H) ; 3,0 (m, 8H) ; 8,0 (sl, 2H) ; 9,0 (s, 3H).
SM-ES⁺ : [M+H]⁺ : 311 ; [M+2H]⁺⁺/2 : 156

### Synthèses

### Dichlorhydrate de 1,12-bis(amidinyl)dodécane: 1.0, 2HCl :

Dans une solution refroidie par un bain de glace à température inférieure à 10°C, de 45 ml d'éthanol anhydre et de 5 g (13 mmol) de chlorhydrate de diéthyltétradécanediimidoate, faire barboter pendant 2 heures, de l'ammoniac gaz. Après quoi, le solvant est évaporé sous pression réduite. Le résidu obtenu est ensuite lavé plusieurs fois avec de l'éther et séché au dessiccateur. On obtient 3,53 g (83%) de produit sous forme de poudre blanche.
oint de fusion: 170-172°C
RMN ¹H (DMSO-d₆, 100 MHz), δ (ppm): 1,25 (s, 16H); 1,58 (m, 4H); 2.37 (t, 4H); 8,82 (s, 4H); 9,10 (s, 4H)
FT-IR, ν ( cm⁻¹): 1688 (C=N); 3081 (NH₂,Cl); 3245 (NH₂)

### 1,12-bis[N,N'-(méthyloxycarbonyl)amidinyl]dodécane: 1.1

A une solution de 1 g (3.06 mmol) de dichlorhydrate de 1,12-bis(amidinyl)dodécane dans 60 ml d'un mélange diphasique dioxane/eau (3:1) et refroidie par un bain de glace, sont ajoutés goutte à goutte et sous agitation vive, 0,60 ml (7,65 mmol) de chloroformiate de méthyle tout en maintenant le pH du mélange entre 10 et 12 avec une solution aqueuse de soude 4N. Le mélange est ensuite laissé sous agitation et à température ambiante durant 3 heures. Après quoi, 100 ml d'eau sont additionnés. Le précipité formé est ensuite essoré, lavé plusieurs fois à l'eau puis à l'éther pour conduire après séchage au dessiccateur, à 1,30 g (77%) de produit sous forme de poudre blanche.
Point de fusion: 116-117°C.
RMN ¹H (DMSO-d₆, 100 MHz), δ (ppm): 1,.69 (s, 16H); 1,96 (m, 4H); 2,61 (t, 4H); 3,97 (s, 6H); 9,06 (s, 4H).
FT-IR, ν (cm⁻¹) : 1256 (C-O-C); 1633 (C=N); 1670 (NHCO); 3183 (NHCO); 3343 (NH)
SM-ES+: [M+H]⁺: 371

### 1,12-bis[N,N'-(éthyloxycarbonyl)amidinyl]dodécane: 1.2

A une solution de 1 g (3,06 mmol) de dichlorhydrate de 1,12-bis(amidinyl)dodécane dans 60 ml d'un mélange diphasique dioxane/eau (3:1) et refroidie par un bain de glace, sont ajoutés goutte à goutte et sous agitation vive, 0,73 ml (7,65 mmol) de chloroformiate d'éthyle tout en maintenant le pH du mélange entre 10 et 12 avec une solution aqueuse de soude 4N. Le mélange est ensuite laissé sous agitation et à température ambiante durant 3 heures. Après quoi, 100 ml d'eau sont additionnés. Le précipité formé est ensuite essoré, lavé plusieurs fois à l'eau puis à l'éther pour conduire après séchage au dessiccateur, à 0.98 g (80%) de produit sous forme de poudre blanche.
Point de fusion: 95-96°C.
RMN ¹H (DMSO-d₆, 100 MHz), δ (ppm): 1.63 (t, 6H) ; 1.71 (m, 16H) ; 1.93 (m, 4H); 2.63 (t, 4H); 4.49 (q, 4H); 8.95 et 9.12 (2s, 4H).
FT-IR, ν (cm⁻¹) : 1251 (C-O-C); 1667 (C=N); 1757 (CO); 3186 (NHCO); 3330 (NH)
SM-ES+: [M+H]⁺: 399; [M+2H]²+/2: 200 (100%)

### 1,12-bis[N,N-(butyloxycarbonyl)amidinyl]dodécane: 1.3

A une solution de 1 g (3,06 mmol) de dichlorhydrate de 1,12-bis(amidinyl)dodécane dans 60 ml d'un mélange diphasique dioxane/eau (3:1) et refroidie par un bain de glace, sont ajoutés goutte à goutte et sous agitation vive, 0,99 ml (7,65 mmol) de chloroformiate de butyle tout en maintenant le pH du mélange entre 10 et 12 avec une solution aqueuse de soude 4N. Le mélange est ensuite laissé sous agitation et à température ambiante pendant 3 heures. Après quoi, 100 ml d'eau sont additionnés. Le précipité formé est ensuite essoré, lavé plusieurs fois à l'eau puis à l'éther pour conduire après séchage au dessiccateur, à 1.12 g (80%) de produit sous forme de poudre blanche.
Point de fusion: 87-88°C.
RMN ¹H (CDCl₃, 100 MHz), δ (ppm): 0.88 (t, 6H); 1.21 (s, 16H) 1.48 - 1.70 (m, 8H); 1.88 (m, 4H); 2.24 (t, 4H); 4.03 (t, 4H); 6.21 (s, 2H); 9.24 (s, 2H).
FT-IR, ν (cm⁻¹): 1084 et 1234 (C-O-C); 1594 (C=N); 1660 (CO); 3313 (NHCO); 3441 (NH)
SM-ES+: [M+H]⁺: 455

### 1,12-bis[N,N'-(isobutyloxycarbonyl) amidinyl] dodécane : 1.4

A une solution de 1 g (3.06 mmol) de dichlorhydrate de 1,12-bis(amidinyl)dodécane dans 60 ml d'un mélange diphasique dioxane/eau (3:1) et refroidie par un bain de glace, sont ajoutés goutte à goutte et sous agitation vive, 1 ml (7.65 mmol) de chloroformiate d'isobutyle tout en maintenant le pH du mélange entre 10 et 12 avec une solution aqueuse de soude 4N. Le mélange est ensuite laissé sous agitation et à température ambiante pendant 3 heures. Après quoi, 100 ml d'eau sont additionnés. Le précipité formé est ensuite essoré, lavé plusieurs fois à l'eau puis à l'éther pour conduire après séchage au dessiccateur, à 1.27 g (91%) de produit sous forme de poudre blanche.
Point de fusion: 102-103°C.
RMN ¹H (CDCl₃, 100 MHz), δ (ppm): 0.92 (d, 12H); 1.22 (s, 16H) 1.62 (m, 4H); 1.96 (m, 2H); 2.26 (t, 4H); 3.82 (d, 4H); 6.19 (s, 2H); 9.27 (s, 2H).
FT-IR, ν (cm⁻¹): 1069 et 1237 (C-O-C); 1599 (C=N); 1661 (CO); 3314 (NHCO); 3440 (NH)
SM-ES+: [M+H]⁺: 455

### 1,12-bis[N,N-(benzyloxycarbonyl)amidinyl]dodécane: 1.5

A une solution de 1 g (3.06 mmol) de dichlorhydrate de 1,12-bis(amidinyl)dodécane dans 60 ml d'un mélange diphasique dioxane/eau (3:1) et refroidie par un bain de glace, sont ajoutés goutte à goutte et sous agitation vive, 1.1 ml (7.65 mmol) de chloroformiate de benzyle tout en maintenant le pH du mélange entre 10 et 12 avec une solution aqueuse de soude 4N. Le mélange est ensuite laissé sous agitation et à température ambiante toute une nuit. Après quoi, 100 ml d'eau sont additionnés. Le précipité formé est ensuite essoré, lavé plusieurs fois à l'eau puis à l'éther pour conduire après séchage au dessiccateur, à 1.2 g (75%) de produit sous forme de poudre blanche.
Point de fusion: 118-119°C.
RMN ¹H (DMSO-d₆, 100 MHz), δ (ppm): 1.19 (s, 16H); 1.47 (m, 4H); 2.13 (t, H); 4.96 (s, 4H); 7.30 (s, 10H); 8.29 (s, 2H); 8.50 (s, 2H).
FT-IR, ν (cm⁻¹): 693 et 745 (C-H aromatique); 1236 (C-O-C); 1648 (C=N); 1666 (CO); 3311 (NHCO); 3436 (NH)
SM-ES+: [M+H]⁺: 523; [M+2H]²+/2: 262 (100%)

### 1,12-bis[N,N'-(4-nitrobenzyloxycarbonyl)amidinyl]dodécane : 1.6

A une solution de 1 g (3.06 mmol) de dichlorhydrate de 1,12-bis(amidinyl)dodécane dans 60 ml d'un mélange diphasique dioxane/eau (3:1) et refroidie par un bain de glace, sont ajoutés goutte à goutte et sous agitation vive, 1.65 g (7.65 mmol) de chloroformiate de 4-nitrobenzyle en solution dans 5 ml de dioxane. Le pH de la solution est maintenu entre 10 et 12 avec une solution aqueuse de soude 4N. Le mélange est ensuite laissé sous agitation et à température ambiante toute une nuit. Après quoi, 100 ml d'eau sont additionnés. Le précipité formé est ensuite essoré, lavé plusieurs fois à l'eau puis à l'éther pour conduire après séchage au dessiccateur, à 1.37 g (74%) de produit sous forme de poudre blanche.
Point de fusion: 87-88°C.
RMN ¹H (DMSO-d₆, 100 MHz), δ (ppm): 1.22 (s, 16H); 1.51 (m, 4H); 2.18 (t, 4H); 5.15 (s, 4H); 7.59 (dd, 4H); 8.23 (dd, 4H); 8.69 (s, 4H).
FT-IR, ν (cm⁻¹) : 1248 (C-O-C); 1344 et 1512 (NO₂); 1621 (C=N); 1658 (CO); 3316 (NHCO); 3406 (NH)
SM-ES+: [M+H]⁺: 623

### 1,12-bis[N,N'-(phényloxycarbonyl)amidinyl]dodécane: 1.7

A une solution de 1.5 g (4.59 mmol) de dichlorhydrate de 1,12-bis(amidinyl)dodécane dans 80 ml d'un mélange diphasique dioxane/eau (3:1) et refroidie par un bain de glace, sont ajoutés goutte à goutte et sous agitation vive, 1.44 ml (11.47 mmol) de phényl chloroformiate tout en maintenant le pH du mélange entre 10 et 12 avec une solution aqueuse de soude 2N. Le mélange est ensuite laissé sous agitation et à température ambiante durant 3 heures. Après quoi, 150 ml d'eau sont additionnés et la solution extraite avec 3 X 60 ml de DCM. La phase organique est ensuite lavée plusieurs fois à l'eau puis évaporée sous pression réduite. On obtient 1.77 g (78%) de produit sous forme liquide.
RMN ¹H (DMSO-d₆, 100 MHz), δ (ppm): 1.73 (s, 16H); 2.05 (m, 4H); 2.72 (t, 4H); 7.17 - 7.90 (m, 10H); 9.20 (s, 4H).
FT-IR, ν (cm⁻¹): 1623 (C=N); 1682 (COO); 3378 (NH et NHCO)
SM-ES+: [M+H]⁺: 495

### 1,12-bis[N,N'-(4-fluorophényloxycarbonyl)amidinyl] dodécane : 1.8

A une solution de 1.5 g (4.59 mmol) de dichlorhydrate de 1,12-bis(amidinyl)dodécane dans 80 ml d'un mélange diphasique dioxane/eau (3:1) et refroidie par un bain de glace, sont ajoutés goutte à goutte et sous agitation vive, 1.50 ml (11.47 mmol) de 4-flurophényl chloroformiate tout en maintenant le pH du mélange entre 10 et 12 avec une solution aqueuse de soude 2N. Le mélange est ensuite laissé sous agitation et à température ambiante durant 3 heures. Après quoi, 150 ml d'eau sont additionnés. Le précipité formé est ensuite essoré, lavé plusieurs fois à l'eau puis à l'éther pour conduire après séchage au dessiccateur, à 1.55 g (64%) de produit sous forme de poudre blanche.
RMN ¹H (CDCl₃, 100 MHz), δ (ppm): 1.26 (s, 16H) ; 1.70 (m, 4H); 2.35 (t, 4H); 6.62 - 7.14 (m, 8H); 6.20 (s, 2H); 9.23 (s, 2H).
FT-IR, ν (cm⁻¹) : 1177 (C-O-C); 1253 (C-F) ; 1622 (C=N); 1679 (COO); 3327 (NH et NHCO)
SM-ES+: [M+H]⁺: 531

### Dichlorhydrate de 1,12-bis [N,N'- (4-fluorophényl-loxycarbonyl)amidinyl] dodécane: 1.8, 2HCl

Dans 20 ml d'une solution éthanolique saturée en acide chlorhydrique gaz, est ajouté 1 g de 1.8. Le mélange réactionnel sous agitation vive est ensuite chauffé à 50°C durant 2 heures. A la solution froide sont additionnés 150 ml d'éther puis le mélange est laissé au repos au réfrigérateur toute une nuit. Après décantation, la couche huileuse formée, est reprise avec 100 ml d'eau distillée puis filtrée. Le filtrat est finalement lyophilisé pour conduire au sel sous forme de poudre blanche.
RMN ¹H (DMSO-d₆, 100 MHz), δ (ppm): 1.75 (s, 16H); 2.15 (m, 4H ); 3.06 (t, 4H); 7.20(s, 2H) ; 7.70 et 7.78 (d, 8H) ; 7.85 et 8.21 (2s, 4H).
FT-IR, ν (cm⁻¹): 1684 (C=N); 1753 (NCO) ; 3324 (NH, HCl).

### 1,12-bis[N,N'-(4-méthoxyphényloxycarbonyl)amidinyl] dodécane: 1.9

A une solution de 1.5 g (4.59 mmol) de dichlorhydrate de 1,12-bis(amidinyl)dodécane dans 80 ml d'un mélange diphasique dioxane/eau (3:1) et refroidie par un bain de glace, sont ajoutés goutte à goutte et sous agitation vive, 1.70 (11.47 mmol) de 4-méthoxyphényl chloroformiate tout en maintenant le pH du mélange entre 10 et 12 avec une solution aqueuse de soude 2N. Le mélange est ensuite laissé sous agitation et à température ambiante durant 3 heures. Après quoi, 150 ml d'eau sont additionnés et la solution extraite avec 3 X 60 ml de DCM. La phase organique est ensuite lavée plusieurs fois à l'eau puis évaporée sous pression réduite. On obtient 1.97 g (78%) de produit sous forme liquide.
RMN ¹H (CDCl₃, 100 MHz), δ (ppm): 1.21 (s, 16H) ; 1.62 (m, 4H); 2.28 (t, 4H); 3.73 (s, 6H) ; 6.71 - 7.06 (m, 8H); 6.18 (s, 2H); 9.21 (s, 2H).
FT-IR, ν (cm⁻¹) : 1176 et 1189 (C-O-C); 1504 (C-H aromatique) ; 1623 (C=N); 1678 (COO); 3374 (NH et NHCO)
SM-ES+: [M+H]⁺: 555

### [1,12-bis(amidinyl)dodécane]-1,12-bis-N,N'-phosphonate de diphényle: 1.12

A une solution de 1.5 g (4.59 mmol) de dichlorhydrate de 1,12-bis(amidinyl)dodécane dans 30 ml d'un mélange diphasique dioxane/eau (3:1) et refroidie par un bain de glace, sont ajoutés goutte à goutte et sous agitation vive, 2.85 ml (13.76 mmol) de diphénylchlorophosphonate tout en maintenant le pH du mélange entre 10 et 12 avec une solution aqueuse de soude 4N. Le mélange est ensuite laissé sous agitation et à température ambiante pendant 3H. Après quoi, 50 ml d'eau sont additionnés. Le précipité formé est ensuite essoré, lavé plusieurs fois à l'eau puis à l'éther pour conduire après séchage au dessiccateur, à 2.70 g (82%) de produit sous forme de poudre blanche.
Point de fusion: 100-101°C
RMN ¹H (DMSO-d₆, 100 MHz), δ (ppm): 1.65 (s, 16H); 1.93 (m, 4H); 2.79 (t, 4H); 7.68 - 7.81 (m, 20H); 8.31 (s, 2H); 8.71 (s, 2H)
RMN ³¹P (DMSO-d₆, 81 MHz), δ (ppm): -1.64
FT-IR, ν (cm⁻¹): 935 (P=O); 1230 (C-O); 1675 (C=N); 3169(NHPO); 3324 (N-H) SM-FAB+: [M+H]⁺: 719; [M+2H]⁺⁺ /2 : 360 (100%)

### [1,12-bis(amidinyl)dodécane]-1,12-bis-N,N'-phosphonate de diéthyle: 1.13

A une solution de 1 g (3.06 mmol) de dichlorhydrate de 1,12-bis(amidinyl)dodécane dans 40 ml d'un mélange diphasique dioxane/eau (3:1) et refroidie par un bain de glace, sont ajoutés goutte à goutte et sous agitation vive, 1.11 ml (7.65 mmol) de diéthylchlorophosphonate tout en maintenant le pH du mélange entre 10 et 12 avec une solution aqueuse de soude 4N. Le mélange est ensuite laissé sous agitation et à température ambiante toute une nuit. Après quoi, la solution est extraite avec du dichlorométhane (3× 30 ml). La phase organique est ensuite lavée plusieurs fois à l'eau puis séchée sur sulfate de sodium. L'évaporation sous pression réduite de la solution conduit à un résidu huileux. Ce dernier repris dans un minimum d'éther et à -4°C toute une nuit, cristallise pour donner 1.08 g (67%) de produit sous forme de poudre blanche.
Point de fusion: 68-69°C.
RMN ¹H (CDCl₃, 100 MHz), δ (ppm): 1.20 (s, 16H); 1.26 (t, 12H); 1.53 (m, 4H); 2.21 (t, 4H); 3.99 (quintuplet, 8H); 6.05 (s, 2H); 7.78 (s, 2H).
RMN ³¹P (CDCl₃, 81 MHz), δ (ppm): 8.76
FT-IR, ν (cm⁻¹) : 793 et 1031 (P-O-CH₂CH₃); 958 (P=O); 1647 (C=N); 3187 (NHPO); 1581et 3386 (N-H).
SM-ES+: [M+H]⁺: 527

### [1,12-bis(amidinyl)dodécane]-1,12-bis-N,N'-phosphonate de sodium: 1.14

### a)acide[1,12-bis(amidinyl)dodécane]-1,12-bis-N,N'-phos-phonique

A une solution de 10 ml de dichlorométhane anhydre refroidie à 0°C sous atmosphère d'azote et sous agitation de 1 g (1.9 mmol) de **1.13,** sont additionnés goutte à goutte 1.36 ml (9.51 mmol) d'iodure de triméthylsilane. Les conditions opératoires sont ainsi maintenues pendant 1 heure. La solution est ensuite évaporée sous pression réduite. Le résidu froid est repris par 10 ml d'acétone contenant 3 ml d'eau puis laissé sous agitation pendant 24 heures. Après quoi, le précipité formé est essoré, lavé plusieurs fois à l'acétone puis recristallisé dans de l'éthanol pour conduire à 0.52 g (66%) de produit sous forme de poudre blanche.
Point de fusion: 164-165°C.
RMN ¹H (DMSO-d₆, 100 MHz), δ (ppm): 1.24 (s, 16H); 1.56 (m, 4H); 2.32 (t, 4H); 9.26 et 9.63 (2s, 8H).
RMN ³¹P (CDCl₃, 81 MHz), δ (ppm): -5.82
FT-IR, ν (cm⁻¹) : 1044 et 1211 (P-OH); 939 (P=O); 1667 (C=N); 2324 (POH); 3019 (NHPO); 1557 et 3322 (N-H).
SM-ES+: [M+H]⁺: 415

### b)[1,12-bis(amidinyl)dodécane]-1,12-bis-N,N'-phosphonate de sodium: 1.14

A une suspension sous agitation de 1 g d' acide[1,12-bis (amidinyl) dodécane]-1,12-bis- *N*,*N*'-phosphonique dans 20 ml d'eau, est additionnée goutte à goutte jusqu'à pH 7.4, une solution de soude 0.1N (environ 21 ml).
Après quoi, la solution est lyophilisée pour conduire à une poudre blanche.
RMN ³¹P (81 MHz), δ (ppm): -3.16

### 1, 12-bis(N,N'-hydroxyamidinyl)dodécane: 1.15

A une solution hydro-alcoolique de soude [préparée à partir de 8.22 g de soude, 36 ml d'eau et 138 ml d'alcool éthylique 95%], sont ajoutés 13.70 g (196.91 mmol) de chlorhydrate d'hydroxylamine. Après 15 mn d'agitation, 20 g (90.91 mmol) de 1,12-dicyanododécane sont additionnés. Le mélange réactionnel est ensuite chauffé à reflux durant 72 heures puis évaporé sous pression réduite. Le résidu obtenu est par suite repris avec de l'eau et laissé sous agitation puis filtré. Le précipité est essoré et lavé plusieurs fois à l'eau et à l'éther de pétrole. Après recristallisation avec de l'éthanol et séchage au dessiccateur toute une nuit, 25 g (96%) de produit sous forme de poudre blanche sont obtenus.
Point de fusion: 170-171 °C.
RMN ¹H (DMSO-d₆, 100 MHz), δ (ppm): 1.70 (s, 16H); 1.90 (m, 4H); 2.39 (t, 4H); 5.75 (s, 4H); 9.13 (s, 2H).
FT-IR, ν (cm⁻¹) : 1661 (C=N); 3244 (N-OH); 3315 et 3400 (NH₂)
+TOF MS: 287 (M + H); 254 (M - 32); 144 (M + H / 2)

### 1, 12-bis(N,N'-méthoxyamidinyl)dodécane: 1.16

A une suspension refroidie par un bain de glace et sous agitation de 2 g (7 mmol) de **1.15** dans 50 ml d'un mélange diphasique dioxane / NaOH 0.7 N, sont ajoutés goutte à goutte 1.70 ml (17.48 mmol) de diméthylsulfate. L'agitation est maintenue toute une nuit à température ambiante. Le mélange réactionnel est ensuite extrait au DCM filtré et le filtrat lavé avec 3 X 100 ml d'eau. La phase organique est séchée sur sulfate de sodium et évaporée sous pression réduite. Le résidu froid cristallise par addition d'éther de pétrole et conduit après séchage au dessiccateur, à 1.05 g (48%) de produit sous forme de poudre blanche.
Point de fusion: 85-86 °C.
RMN ¹H (CDCl₃, 100 MHz), δ (ppm): 1.23 (s, 16H); 1.50 (m, 4H); 2.10 (t, 4H); 3.74 (s, 6H). 4.44 (s, 4H).
FT-IR, ν (cm⁻¹) : 1048 (N-O-C) ; 1643 (C=N); 3288 et 3433 (NH₂) +TOF MS: 315 (M + H)

### 1, 12-bis(N,N'-éthoxycarbonyloxyamidinyl)dodécane: 1.17

A une suspension sous agitation de 2 g (7 mmol) de 1.15 et de 2.65 ml de triéthylamine (18.9 mmol) dans 45 ml de chloroforme, sont ajoutés goutte à goutte 1.40 ml (14.68 mmol) du chloroformiate d'éthyle dans 5 ml de chloroforme. L'agitation est maintenue durant 3 heures à température ambiante. Le mélange réactionnel est ensuite filtré et le filtrat lavé avec 3 X 100 ml d'eau. La phase organique est séchée sur sulfate de sodium et évaporée sous pression réduite. Après cristallisation du résidu froid, les cristaux sont lavés à l'éther de pétrole et séchés au dessiccateur pour conduire à 2.53 g (84.33%) de produit sous forme de poudre blanche.
Point de fusion: 100-101 °C.
RMN ¹H (CDCl₃, 100 MHz), δ (ppm): 1.21 (s, 16H); 1.29 (t, 6H) 1.53 (m, 4H); 2.19 (t, 4H); 4.23 (quartet, 4H). 4.79 (s, 4H).
FT-IR, ν (cm⁻¹) : 1240 (C-O-C); 1620 (C=N); 1741 (OCOO); 3374 et 3508 (NH₂) +TOF MS: 431 (M + H)

### 1, 12-bis(N,N'-méthoxycarbonyloxyamidinyl)dodécane: 1.18

A une suspension sous agitation de 2 g (7 mmol) de 1.15 et de 2.65 ml de triéthylamine (18.9 mmol) dans 45 ml de chloroforme, sont ajoutés goutte à goutte 1.14 ml (14.68 mmol) de chloroformiate de méthyle dans 5 ml de chloroforme. L'agitation est maintenue durant 3 heures à température ambiante. Le mélange réactionnel est ensuite filtré et le filtrat lavé avec 3 X 100 ml d'eau. La phase organique est séchée sur sulfate de sodium et évaporée sous pression réduite. Le résidu froid est lavé avec de l'éther de pétrole puis essoré et séché au dessiccateur pour conduire à 2.05 g (73%) de produit sous forme de poudre blanche.
Point de fusion: 79-80 °C.
RMN ¹H (CDCl₃, 100 MHz), δ (ppm): 1.21 (s, 16H); 1.53 (m, 4H); 2.19 (t, 4H); 3.81 (s, 6H). 4.81 (s, 4H).
FT-IR, ν (cm⁻¹) : 1247 (C-O-C); 1622 (C=N); 1750 (OCOO); 3380 et 3497 (NH₂) +TOF MS: 403 (M + H)

### 1, 12-bis(N,N'-phénoxycarbonyloxyamidinyl)dodécane: 1.19

A une suspension sous agitation de 2 g (7 mmol) de 1.15 et de 2.65 ml de triéthylamine (18.9 mmol) dans 30 ml de DMF et refroidie par un bain d'eau froid, sont ajoutés goutte à goutte 1.85 ml (14.68 mmol) chloroformiate de phényle. L'agitation est maintenue durant 4 heures à température ambiante puis le mélange réactionnel est filtré et le filtrat dilué avec 150 ml d'acétate d'éthyle. La solution est ensuite lavée avec de l'eau (100 ml) et avec une solution saturée de chlorure de sodium (2 X 100 ml). La phase organique est séchée sur sulfate de sodium et évaporée sous pression réduite. Le résidu froid est lavé avec de l'éther de pétrole puis essoré et séché au dessiccateur pour conduire à 3.02 g (82%) de produit sous forme de poudre blanche.
Point de fusion: 108-109 C.
RMN ¹H (DMSO-d₆, 100 MHz), δ (ppm): 1.72 (s, 16H); 1.98 (m, 4H); 2.52 (t, 4H); 6.98 (s, 4H); 7.65 - 7.98 (m, 10H).
FT-IR, ν (cm⁻¹) : 1197 et 1241 (C-O-C); 1633 (C=N); 1770(OCOO); 3309 et 3473 (NH₂)
+TOF MS: (M + H)

### 1,12-bis(N,N'-thiométhylcarbonyloxyamidinyl)dodécane: 1.20

A une suspension sous agitation de 2.46 g (8.60 mmol) de 1.15 et de 3.26 ml de triéthylamine (23.22 mmol) dans 45 ml de chloroforme, sont ajoutés goutte à goutte 1.55 ml (18.06 mmol) de chloroformiate de thiométhyle dans 5 ml de chloroforme. L'agitation est maintenue durant 2 heures à température ambiante. Le mélange réactionnel est ensuite filtré et le filtrat lavé avec 3 X 100 ml d'eau. La phase organique est séchée sur sulfate de sodium et évaporée sous pression réduite. Le résidu froid est lavé avec de l'éther de pétrole puis essoré et séché au dessiccateur pour conduire à 3.45g (92%) de produit sous forme de poudre blanche.
Point de fusion: 89-90 °C.
RMN ¹H (CDCl₃, 100 MHz), δ (ppm): 1.24 (s, 16H); 1.56 (m, 4H); 2.20 (t, 4H); 2.31 (s, 6H). 4.79 (s, 4H).
FT-IR, n (cm⁻¹) : 1131 (C-O-C); 1605 (C=N); 1719 (OCOS); 3384 et 3496 (NH₂) ES+ MS: 435 (M + H)

### 1,12-bis(N,N'-thioéthylcarbonyloxyamidinyl)dodécane: 1.21

A une suspension sous agitation de 2 g (7 mmol) de 1.15 et de 2.65 ml de triéthylamine (18.9 mmol) dans 45 ml de chloroforme, sont ajoutés goutte à goutte 1.53 ml (14.68 mmol) de chloroformiate de thioéthyle dans 5 ml de chloroforme. L'agitation est maintenue durant 3 heures à température ambiante. Le mélange réactionnel est ensuite filtré et le filtrat lavé avec 3 X 100 ml d'eau. La phase organique est séchée sur sulfate de sodium et évaporée sous pression réduite. Le résidu froid est lavé avec de l'éther de pétrole puis essoré et séché au dessiccateur pour conduire à 2.70 g (83%) de produit sous forme de poudre blanche.
Point de fusion: 59-60 °C.
RMN ¹H (CDCl₃, 100 MHz), δ (ppm): 1.23 (s, 16H); 1.30 (t, 6H) 1.56 (m, 4H); 2.20 (t, 4H); 2.86 (quartet, 4H). 4.80 (s, 4H).
FT-IR, n (cm⁻¹) : 1127 (C-O-C); 1608 (C=N); 1718 (OCOS); 3386 et 3496 (NH₂) ES+ MS: 462 (M + H)

### 1, 12-bis(N,N'-acétoxyamidinyl)dodécane: 1.22

A 26 ml (280 mmol) d'anhydride acétique sous agitation et refroidie par un bain d'eau glacée, sont ajoutés portion par portion 2 g (7 mmol) de 1.15. L'agitation est maintenue durant 2 heures à température ambiante. Au mélange réactionnel, sont ajoutés 100 ml de chloroforme. La solution est ensuite lavée successivement avec 2 X 100 ml d'une solution aqueuse saturée de chlorure de sodium, 3 X 100 ml d'une solution de soude 3N et 100 ml d'eau. La phase organique est séchée sur sulfate de sodium et évaporée sous pression réduite. Après cristallisation du résidu froid, les cristaux sont lavés à l'éther de pétrole et séchés au dessiccateur pour conduire à 2.33 g (90%) de produit sous forme de poudre blanche.
Point de fusion: 128-129 °C.
RMN ¹H (CDCl₃, 100 MHz), δ (ppm): 1.23 (s, 16H); 1.55 (m, 4H); 2.12 (s, 6H); 2.23 (t, 4H); 5.74 (s, 4H).
FT-IR, ν (cm⁻¹) : 1232 (C-O-C); 1633 (C=N); 1736 (OCO); 3319 et 3425 (NH₂)
+TOF MS: 371 (M + H)

### 1, 12-bis(N,N'-benzoyloxyamidinyl)dodécane: 1.23

A une suspension sous agitation de 1.5 g (5.24 mmol) de 1.15 et de 2 ml de triéthylamine (14.16 mmol) dans 30 ml de DMF et refroidie par un bain d'eau froid, sont ajoutés goutte à goutte 1.28 ml (14.68 mmol) chlorure de benzoyle. L'agitation est maintenue durant 3 heures à température ambiante puis le mélange réactionnel est filtré et le filtrat précipité dans 200 ml d'eau froide. Le précipité est ensuite essoré, lavé à l'eau et à l'éther de pétrole puis séché au dessiccateur pour conduire à 2.25 g (87%) de produit sous forme de poudre blanche.
Point de fusion: 158-159 C.
RMN ¹H (DMSO-d₆, 100 MHz), δ (ppm): 1.73 (s, 16H); 2.01 (m, 4H); 2.56 (t, 4H); 6.94 (s, 4H); 7.96 - 8.11 (m, 6H); 8.54 - 8.61 (m, 4H).
FT-IR, ν (cm⁻¹) : 684 et 699 (C-H aromatiques); 1266 (C-O-C); 1625 (C=N); 1721 (OCO); 3315 et 3452 (NH₂)

### 1, 12-bis(N,N'-éthylcarbamoyloxyamidinyl)dodécane: 1.24

A une suspension sous agitation de 2 g (7 mmol) de 1.15 et de 1.01 g de carbonate de potassium (7.34 mmol) dans 80 ml de chloroforme, sont ajoutés goutte à goutte 1.16 ml (14.68 mmol) d'éthyle isocyanate. L'agitation est maintenue toute une nuit à température ambiante. Le mélange réactionnel est ensuite filtré et le filtrat lavé avec 2 X 100 ml d'eau. La phase organique est par suite séchée sur sulfate de sodium et évaporée sous pression réduite pour conduire à 2.22 g (74%) de produit sous forme d'huile colorée.
RMN ¹H (CDCl₃, 100 MHz), δ (ppm): 1.14 (t, 6H); 1.22 (s, 16H); 1.51 (m, 4H); 2.12 (t, 4H); 3.26 (quintuplet, 4H). 5.00 (s, 4H); 6.44 (t, 2H).
FT-IR, ν (cm⁻¹) : 1650 (C=N); 1701 (OCONH); 3335 (NH); 3374 et 3490 (NH₂) +TOF MS: 429 (M + H)

### 1,12-bis(N,N'-phénylcarbamoyloxyamidinyl)dodécane: 1.25

A une suspension sous agitation refroidie à 0°C par un bain d'eau froid, de 2 g (7 mmol) de **1.15** et de 1.01 g de carbonate de potassium (7.34 mmol) dans 40 ml de DMF, sont ajoutés goutte à goutte 1.6 ml (14.68 mmol) de phényle isocyanate. L'agitation est maintenue durant 2H30 à température ambiante. Le mélange réactionnel est ensuite filtré et le filtrat précipité dans 150 ml d'eau froide. Le précipité est par suite lavé successivement avec de l'eau, de l'acétone et de l'éther. Après séchage au dessiccateur, 2.95 g (80%) de produit sont obtenus sous forme de poudre blanche.
Point de fusion: 134-135 °C.
RMN ¹H (DMSO-d₆, 100 MHz), δ (ppm): 1.72 (s, 16H); 2.0 (m, 4H); 2.55 (t, 4H); 6.83 (s, 4H); 7.54 (t, 2H); 7.76 (t, 4H); 7.93 (t, 4H); 9.65 (s, 2H).
FT-IR, ν (cm⁻¹) : 1018 et 1220 (C-O-C); 1628 (C=N); 1708 (CON); 3249 (OCONH); 3345 et 3455 (NH₂)
ES+ MS: 525 (M + H); 263 (M + H/2)

### [1,12-bis(amidinyl)dodécane]-1,12-bis-N,N'-phosphate de diéthyle: 1.26

A une suspension sous agitation refroidie à 0°C par un bain d'eau froid, de 2 g (7 mmol) de **1.15** et de 2.06 ml de triéthylamine (14.68 mmol) dans 30 ml de DMF, sont ajoutés goutte à goutte 2.08 ml (14.33 mmol) de diéthylchlorophosphonate. L'agitation est maintenue durant 16H à température ambiante. Le mélange réactionnel est ensuite filtré et le filtrat repris avec 150 ml d'acétate d'éthyle. La phase organique est par suite lavée avec 3 X 200 ml d'eau puis séchée sur sulfate de sodium et évaporée sous pression réduite pour conduire à 2.88 g (74%) de produit sous forme d'huile colorée.
RMN ¹H (CDCl₃, 100 MHz), δ (ppm): 1.21 (s, 16H); 1.31 (t, 12H); 1.51 (m, 4H); 2.15 (t, 4H); 4.15 (quintuplet, 8H); 4.30 (s, 4H).
FT-IR, ν (cm⁻¹) : 837 et 1027 (OP-O-CH₂CH₃); 969 (OP=O); 1648 (C=N); 3323 et 3487 (NH₂).
ES+ MS: 559 (M + H)

### 1,12-bis[(1,2,4-oxadiazol-5(4H)-one)-3-yl]dodécane: 1.27

Une suspension sous agitation de 4 g (9.30 mmol) de 1.17 dans 70 ml de xylène, est chauffée à 150°C durant 2 heures (jusqu'à la formation d'une couche huileuse colorée). Le mélange réactionnel est ensuite évaporé sous pression réduite. Le résidu solide obtenu est par suite dissout dans 50 ml de DMSO puis précipité dans 200 ml d'eau froide. Le précipité formé est essoré puis redissout dans de l'acétone et filtré. Le filtrat séché sur sulfate de sodium et évaporé sous pression réduite conduit après séchage au dessiccateur, à 2.97 g (94%) de produit sous forme de cristaux colorés.
Point de fusion: 150-151 °C.
RMN ¹H (DMSO-d₆, 100 MHz), δ (ppm): 1.71 (s, 16H); 2.03 (m, 4H); 2.93 (t, 4H).
FT-IR, n (cm⁻¹) : 1718 (C=N); 1783 (OCONH); 3163 (NHCO).
ES+ MS: 339 (M + H)

### 1,12-bis[(1,2,3,5-oxathiadiazol-2(3H)-oxyde)-4-yl] dodécane : 1.28

A une suspension sous agitation et refroidie à 0°C de 2 g (7 mmol) de 1.15 et de 2.82 ml de pyridine (34.96 mmol) dans 30 ml de DMF, sont ajoutés goutte à goutte 1.1 ml (15.03 mmol) de chlorure de thionyle. L'agitation est maintenue durant 45 minutes à froid puis la solution réactionnelle est précipitée dans 150 ml d'eau froide. Le précipité filtré et séché au dessiccateur, conduit à 1.65 g (62%) de produit sous forme de poudre colorée.
Point de fusion: 94-95 °C.
RMN ¹H (DMSO-d₆, 100 MHz), δ (ppm): 1.71 (s, 16H); 2.05 (m, 4H); 2.99 (t, 4H).
FT-IR, n (cm⁻¹) : 1146 (NSOC); 1615 (C-N); 1655 (C=N); 3219 et 3323 (NHSO).
ES+ MS: 379 (M + H)

### 1,12-bis[(1,2,4-oxadiazol-5(4H)-thione)-3-yl]dodécane: 1.29

A une suspension sous agitation et refroidie à 0°C de 2 g (5.4 mmol) de 1.22 et de 2.5 ml (34.96 mmol) de sulfure ce carbone dans 50 ml de DMF, sont ajoutés lentement 1.32 g (32.97 mmol) d'hydrure de sodium 60%. L'agitation est maintenue durant 45 minutes à froid puis durant 2 heures à température ambiante. La solution réactionnelle est ensuite précipitée dans 150 ml d'eau froide, acidifiée à pH 5 avec HCl 2N puis extraite avec 3 X 50 ml d'acétate d'éthyle. La phase organique lavée à l'eau puis séchée sur sulfate de sodium et évaporée sous pression réduite conduit à un résidu solide. Ce dernier est ensuite lavé avec de l'éther pour donner lieu après séchage au dessiccateur à 1.45 g (72%) de produit sous forme de poudre coloré.
RMN ¹H (DMSO-d₆, 100 MHz), δ (ppm): 1.71 (s, 16H); 2.06 (m, 4H); 3.02 (t, 4H); 9.25 (s, 2H)
FT-IR, ν (cm⁻¹) : 1644 (C=N); 1737 (SCONH); 3090 (NHCO).
ES+ MS: 371 (M + H); 313 [M + 2H - 60 (SCO)]

### 1,12-bis[(1,2,4-thiadiazol-5(4H)-one)-3-yl]dodécane: 1.30

Une suspension de 2 g (7 mmol) de 1.15 et de 3.74 g (21 mmol)de 1,1'-thiocarbonyl diimidazole dans 70 ml de THF est laissée sous agitation et à température ambiante durant 16 heures. Le mélange réactionnel est dilué avec 150 ml d'eau et extrait avec 3 X 70 ml d'acétate d'éthyle. La phase organique est ensuite lavée avec de l'eau, séchée sur sulfate de sodium et évaporée sous pression réduite. Le résidu huileux est repris avec 50 ml de THF auquel sont ajoutés 5.32 ml (41.96 mmol) de BF₃, Et₂O. Le mélange réactionnel obtenu est par suite laissé sous agitation toute une nuit à température ambiante. La solution est diluée avec de l'eau, extraite avec de l'acétate d'éthyle, lavée à l'eau, séchée puis évaporée sous pression réduite. Le résidu froid obtenu est ensuite repris avec 50ml d'éthanol et précipité dans 200 ml d'eau froide. Le précipité isolé, est finalement lavé à l'eau et séché pour donner 1.62 g (62.55%) de produit sous forme de poudre orange.
RMN ¹H (DMSO-d₆, 100 MHz), δ (ppm): 1.70 (s, 16H); 2.04 (m, 4H); 2.93 (t, 4H); 9.30 (s, 2H)
FT-IR, ν (cm⁻¹): 1666 (C=N); 1707 (SCONH); 3129 (NHCO).
+TOF MS: 313 [M + 2H - 60 (SCO)]

### 1,14-di(pyrrolidin-1-yl)tétradécane-1,14-diimine: 2.0, 2HCl

Un mélange constitué de 1,16 g (3 mmol) de dichlorhydrate de diéthyl tétradécanediimidoate, 0,51 ml de pyrrolidine et 15 ml d'éthanol est chauffé au reflux pendant 24 heures. Après évaporation, le résidu obtenu est recristallisé dans un mélange méthanol-éther pour conduire à 0,97 g (75%) de poudre beige.
Point de fusion: 171°C
RMN ¹H (CD₃OD, 250 MHz), δ (ppm): 1,35 (m, 16H); 1,68 (m, 4H); 2,08
(m, 8H); 2,55 (t, 4H); 3,42 (t, 4H); 3,70 (t, 4H); 9,12 (s, 4H)

### 1,14-di(pipéridin-1-yl)tétradécane-1,14-diimine: 3.0

On ajoute à 2,2 g (10 mmol) de 1,12-dicyanododécane et 1,98 g (20 mmol) de CuCl, 1,68 ml (20 mmol) de pipéridine anhydre. Le mélange, initialement vert, bleuit. On chauffe ensuite à 80°C pendant 20 heures, et la solution rouge résultante est versée dans 125 ml d'éther et agitée 2 mn avec 12 ml de NaOH (30% aqueux). La phase organique est isolée et séchée sur SO₄Na₂, filtrée et évaporée. Le résidu obtenu est recristallisé dans l'éther (Rendement 50%).
Point de fusion: 149-150°C
RMN ¹H (CDCl₃, 100 MHz), δ (ppm): 1,3 (m, 20H); 1,4-1,6 (m, 12H); 2,2 (t, 4H); 3,3 (m, 8H); 6,5 (m, 2H).

### N¹,N¹,N¹⁴,N¹⁴-tétraméthyltétradécanediimidamide: 4.0, 2HCl

1,16 g (3 mmol) de dichlorhydrate de diéthyl tétradécanediimidoate et 1,1 ml d'une solution de diméthylamine dans l'éthanol (5,6M) sont mélangés à 15 ml d'éthanol et chauffés au reflux pendant 26 heures. Après quoi, le solvant est évaporé et le résidu obtenu est recristallisé dans un mélange méthanol-éther pour conduit à 0,98 g (85%) de poudre verdâtre.
Point de fusion: 209°C
RMN ¹H (CD₃OD, 250 MHz), δ (ppm): 1,35 (m, 16H); 1,68 (m, 4H); 2,59
(t, 4H); 3,12 (s, 6H); 3,25 (s, 6H); 9,10 (s, 4H)

### N¹,N¹,N¹⁴,N¹⁴-tétraméthyloctadécanediimidamide: 5.0

Un mélange de 0,46 g (1,2 mmol) de dichlorhydrate de diéthyl octadécanediimidoate dans 5,3 ml d'une solution d'ammoniac dans le méthanol (2M) est refroidie à -10°C pendant 1 heure, puis laissée à température ambiante 15 heures. Après évaporation du solvant, le résidu obtenu est recristallisé à l'aide d'un mélange méthanol-éther, filtré et séché au dessiccateur, pour conduire à 0,25 g (54%) de produit.
Point de fusion: 196-198°C
RMN 1^{E}H (DMSO-d₆, 250 MHz), δ (ppm): 1,24 (m, 24H); 1,60 (m, 4H); 2.37 (t, 4H); 8.82 (s, 4H); 9.10 (s, 4H)

### 1,12-bis(imidazolin-2-yl)dodécane: 6.0

### a) Dichlorhydrate de 1,12-bis(imidazolin-2-yl)dodécane: 6.0, 2HCl

A une solution de 4.5 g (11.69 mmol) de chlorhydrate de 1,14-diéthoxytétradécane-1,14-diimine dans 100 ml d'éthanol absolu, sont ajoutés lentement et à froid 1.64 ml (24.55 mmol) d'éthylène diamine. Le mélange réactionnel est ensuite chauffé à reflux pendant 4 heures. Après évaporation sous pression réduite au minimum de solvant, le résidu froid obtenu est cristallisé par addition d'éther sous agitation. Le précipité isolé par filtration, est ensuite lavé à l'éther et séché au dessiccateur, pour conduire à 3.9 g (88%) de produit sous forme de poudre blanche.
Point de fusion: 191-192°C
RMN ¹H (DMSO-d₆, 100 MHz), δ (ppm): 1.22 (s, 16H); 1.57 (m, 4H); 2.45 (t, 4H); 3.75 (s, 8H); 9.28 (s, 4H).
FT-IR, ν (cm⁻¹): 1600 (C=N); 3031 (C=NH, HCl); 3200 (N-H)

### b) 1,12-bis(imidazolin-2-yl)dodécane: 6.0

A une solution aqueuse (20 ml) de 2 g (5.28 mmol) de 6.0, 2 HCl , est additionnée goutte à goutte et sous agitation, de la triéthylamine jusqu'à pH 14. Le précipité formé est essoré, lavé à l'eau, à l'acétone puis à l'éther et séché au dessiccateur pour conduire à 1.4 g (86.4%) de base libre sous forme de poudre blanche. Cette poudre est ensuite recristallisée dans du méthanol et donne 1.26 g (90%) de produit sous forme de cristaux blancs.
Point de fusion: 176-178°C
RMN ¹H (CD₃OD, 100 MHz), δ (ppm): 1.29 (s, 16H); 1.55 (m, 4H); 2.20 (t, 4H); 3.52 (s, 8H).
FT-IR, ν (cm⁻¹): 1611 (C=N); 3177 (N-H)

### 1,12-bis[N,N'-(méthyloxycarbonyl)imidazolin-2-yl] dodécane : 6.1

A une suspension de 1.5 g (4.90 mmol) de 1,12-bis(imidazolin-2-yl)dodécane et de 1.5 ml (10.78 mmol) de triéthylamine dans 25 ml de chloroforme refroidie par un bain de glace, sont additionnés goutte à goutte et sous agitation 0.80 ml (10.29 mmol) de chloroformiate de méthyle en solution dans 5 ml de chloroforme. Après quoi, l'agitation est maintenue à température ambiante toute une nuit Le mélange réactionnel est ensuite filtré et le filtrat lavé successivement avec 120 ml d'eau, 120 ml d'une solution aqueuse saturée en chlorure de sodium et 2 x 120 ml d'eau. Après séchage sur sulfate de sodium, la phase organique est évaporée sous pression réduite pour conduire à un résidu huileux. Ce dernier repris dans de l'éther de pétrole et laissé à - 4°C donne 1.51 g (73%) de produit sous forme d'un liquide blanc.
RMN ¹H (CDCl₃, 100 MHz), δ (ppm): 1.20 (s, 16H); 1.58 (m, 4H); 2.64 (t, 4H); 3.70 (s, 8H); 3.76 (s, 6H).
FT-IR, ν (cm⁻¹): 1142 et 1194 (C-O-C); 1642 (C=N); 1723 (NCO).

### 1,12-bis[N,N'-(éthyloxycarbonyl)imidazolin-2-yl]dodécane : 6.2

A une suspension de 1.5 g (4.90 mmol) de 1,12-bis(imidazolin-2-yl)dodécane et de 1.5 ml (10.78 mmol) de triéthylamine dans 25 ml de chloroforme refroidie par un bain de glace, sont additionnés goutte à goutte et sous agitation 0.98 ml (10.29 mmol) de chloroformiate d'éthyle en solution dans 5 ml de chloroforme. Après quoi, l'agitation est maintenue à température ambiante toute une nuit. Le mélange réactionnel est ensuite filtré et le filtrat lavé successivement avec 120 ml d'eau, 120 ml d'une solution aqueuse saturée en chlorure de sodium et 2 x 120 ml d'eau. Après séchage sur sulfate de sodium, la phase organique est évaporée sous pression réduite pour conduire à 1.78 g (81%) de produit sous forme d'huile.
RMN ¹H (CDCl₃, 100 MHz), δ (ppm): 1.22 (s, 16H); 1.30 (t, 6H) ; 1.59 (m, 4H); 2.65 (t, 4H); 3.72 (s, 8H); 4.16 (q, 4H).
FT-IR, ν (cm⁻¹): 1073 et 1099 (C-O-C); 1644 (C=N); 1720 (NCO).

### 1,12-bis[N,N'-(butyloxycarbonyl)imidazolin-2-yl]dodécane : 6.3

A une suspension de 1.5 g (4.90 mmol) de 1,12-bis(imidazolin-2-yl)dodécane et de 1.5 ml (10.78 mmol) de triéthylamine dans 25 ml de chloroforme refroidie par un bain de glace, sont additionnés goutte à goutte et sous agitation 1.33 ml (10.29 mmol) de chloroformiate de butyle en solution dans 5 ml de chloroforme. Après quoi, l'agitation est maintenue à température ambiante toute une nuit. Le mélange réactionnel est ensuite filtré et le filtrat lavé successivement avec 120 ml d'eau, 120 ml d'une solution aqueuse saturée en chlorure de sodium et 2 x 120 ml d'eau. Après séchage sur sulfate de sodium, la phase organique est évaporée sous pression réduite pour conduire à 2.05 g (82%) de produit sous forme d'huile.
RMN ¹H (CDCl₃, 100 MHz), δ (ppm): 0.88 (t, 6H) ; 1.20 (s, 16H); 1.36 -1.64 (m, 12H); 2.63 (t, 4H); 3.71 (s, 8H); 4.08 (t, 4H).
FT-IR, ν (cm⁻¹): 1073 et 1152 (C-O-C); 1644 (C=N); 1722 (NCO).

### 1,12-bis[N,N'-(isobutyloxycarbonyl)imidazolin-2-yl] dodécane : 6.4

A une suspension de 1.5 g (4.90 mmol) de 1,12-bis(imidazolin-2-yl)dodécane et de 1.5 ml (10.78 mmol) de triéthylamine dans 25 ml de chloroforme refroidie par un bain de glace, sont additionnés goutte à goutte et sous agitation 1.35 ml (10.29 mmol) de chloroformiate d'isobutyle en solution dans 5 ml de chloroforme. Après quoi, l'agitation est maintenue à température ambiante toute une nuit. Le mélange réactionnel est ensuite filtré et le filtrat lavé successivement avec 120 ml d'eau, 120 ml d'une solution aqueuse saturée en chlorure de sodium et 2 x 120 ml d'eau. Après séchage sur sulfate de sodium, la phase organique est évaporée sous pression réduite pour conduire à un résidu huileux. Ce dernier repris dans de l'éther de pétrole et laissé à -4°C donne 2.26 g (91 %) de produit sous forme d'un solide blanc.
Point de fusion: < 40°C
RMN ¹H (CDCl₃, 100 MHz), δ (ppm): 0.87 (d, 12H) ; 1.17 (s, 16H); 1.55 (m, 4H) ; 1.89 (quint., 2H); 2.62 (t, 4H); 3.70 (s, 8H); 3.84 (d, 4H).
FT-IR, ν (cm⁻¹): 1072 et 1142 (C-O-C); 1642 (C=N); 1722 (NCO).

### 1,12-bis[N,N'-(benzyloxycarbonyl)imidazolin-2-yl] dodécane : 6.5

A une suspension de 1 g (3.27 mmol) de 1,12-bis(imidazolin-2-yl)dodécane et de 0.85 ml (6.54 mmol) de triéthylamine dans 30 ml de chloroforme refroidie par un bain de glace, sont additionnés goutte à goutte et sous agitation 0.94ml (6.54 mmol) de chloroformiate de benzyle dans 5 ml de chloroforme. Après quoi, l'agitation est maintenue à température ambiante toute une nuit. La solution obtenue est évaporée sous pression réduite. Le précipité isolé est par suite extrait plusieurs fois avec de l'éther. L'évaporation sous pression réduite de la phase éthérée, conduit à 1.02 g (54%) de produit sous forme d'huile se cristallisant en poudre blanche à - 4°C dans de l'éther.
Point de fusion: 75-76°C
RMN ¹H (CDCl₃, 100 MHz), δ (ppm): 1.23 (s, 16H); 1.60 (m, 4H); 2.68 (t, 4H); 3.77 (s, 8H); 5.16 (s, 4H); 7.35 (s, 10H).
FT-IR, ν (cm⁻¹) : 1142 et 1299 (C-O-C); 1646 (C=N); 1714 (NCO)
SM-ES+: [M+H]⁺: 575

### 1,12-bis[N,N'-(4-nitrobenzyloxycarbonyl)imidazolin-2-yl]dodécane : 6.6

A une suspension de 1 g (3.27 mmol) de 1,12-bis(imidazolin-2-yl)dodécane et de 0.87 ml (6.70 mmol) de triéthylamine dans 20 ml de chloroforme refroidie par un bain de glace, sont additionnés goutte à goutte et sous agitation 1.44 g (6.70 mmol) de chloroformiate de 4-nitrobenzyle en solution dans 10 ml de chloroforme. Après quoi, l'agitation est maintenue à température ambiante toute une nuit. Le mélange réactionnel est ensuite dilué avec 30 ml de chloroforme puis lavé successivement avec 120 ml d'eau, 120 ml d'une solution aqueuse saturée en chlorure de sodium et 2 x 120 ml d'eau. Après séchage sur sulfate de sodium, la phase organique est évaporée sous pression réduite. Le résidu refroidi et repris dans un minimum de chloroforme, est enfin cristallisé à -4°C avec de l'hexane pour conduire à 2.08g (96%) de produit sous forme de poudre blanche.
Point de fusion: 115-116°C
RMN ¹H (CDCl₃, 100 MHz), δ (ppm): 1.21 (s, 16H); 1.60 (m, 4H); 2.67 (t, 4H); 3.80 (s, 8H); 5.24 (s, 4H); 7.46 et 7.55 (dd, 4H); 8.17 et 8.25 (dd, 4H).
FT-IR, ν (cm⁻¹): 1005 et 1154 (C-O-C); 1343 et 1517 (NO₂); 1645 (C=N); 1724 (NCO).
SM-ES+: [M+H]⁺: 665

### 1,12-bis[N,N'-(phényloxycarbonyl)imidazolin-2-yl] dodécane : 6.7

A une suspension de 1.5 g (4.90 mmol) de 1,12-bis(imidazolin-2-yl)dodécane et de 1.3 ml (10.05 mmol) de triéthylamine dans 25 ml de chloroforme refroidie par un bain de glace, sont additionnés goutte à goutte et sous agitation 1.26 (10.05 mmol) de chloroformiate de phényle en solution dans 5 ml de chloroforme. Après quoi, l'agitation est maintenue à température ambiante toute une nuit. Le mélange réactionnel est ensuite dilué avec 30 ml de chloroforme puis lavé successivement avec 120 ml d'eau, 120 ml d'une solution aqueuse saturée en chlorure de sodium et 2 x 120 ml d'eau. Après séchage sur sulfate de sodium, la phase organique est évaporée sous pression réduite pour conduire à 2.42 g (90%) de produit sous forme d'huile.
RMN ¹H (CDCl₃, 100 MHz), δ (ppm): 1.21 (s, 16H); 1.64 (m, 4H); 2.72 (t, 4H); 3.89 (s, 8H); 7.06 - 7.43 (m, 10H)
FT-IR, ν (cm⁻¹): 1162 et 1188 (C-O-C); 1646 (C=N); 1737 (NCO).
SM-ES+: [M+H]⁺: 547; [M+2H]²+/2 : 274 (100%)

### 1,12-bis[N,N'-(4-fluorophénylyloxycarbonyl)imidazolin-2-yl]dodécane: 6.8

A une suspension de 1 g (3.27 mmol) de 1,12-bis(imidazolin-2-yl)dodécane et de 0.89 ml (6.86 mmol) de triéthylamine dans 20 ml de chloroforme refroidie par un bain de glace, sont additionnés goutte à goutte et sous agitation 0.89 ml (6.70 mmol) de chloroformiate de 4-fluorophényle en solution dans 5 ml de chloroforme. Après quoi, l'agitation est maintenue à température ambiante toute une nuit. Le mélange réactionnel est ensuite dilué avec 20 ml de chloroforme puis lavé successivement avec 60 ml d'eau, 60ml d'une solution aqueuse saturée en chlorure de sodium et 2 x 60 ml d'eau. Après séchage sur sulfate de sodium, la phase organique est évaporée sous pression réduite. Le résidu refroidi est enfin cristallisé à -4°C avec de l'hexane pour conduire à 1.70 g (85%) de produit sous forme de poudre blanche.
Point de fusion: 95-96°C
RMN ¹H (CDCl₃, 100 MHz), δ (ppm): 1.21 (s, 16H); 1.64 (m, 4H); 2.71 (t, 4H); 3.90 (s, 8H); 7.03 et 7.36 (d, 8H).
FT-IR, ν (cm⁻¹): 1100 (C-F); 1179 (C-O-C); 1655 (C=N); 1733 (NCO).
SM-ES+: [M+H]⁺: 583; [M+2H]²+/2 : 292 (100%)
Dichlorhydrate de 1,12-bis[N,N'-(4-fluorophénylyl oxycarbonyl)imidazolin-2-yl]dodécane: 6.8, 2 HCl.

Dans 20 ml d'une solution éthanolique saturée en acide chlorhydrique gaz, est ajouté 1 g de 6.8. Le mélange réactionnel sous agitation vive est ensuite chauffé à 50°C durant 4 heures. A la solution froide sont additionnés 150 ml d'éther puis le mélange est laissé au repos au réfrigérateur toute une nuit. Après décantation, la couche huileuse formée, est reprise avec 100 ml d'eau distillée puis filtrée. Le filtrat est finalement lyophilisé pour conduire au sel sous forme de poudre blanche.
RMN ¹H (DMSO-d₆, 100 MHz), δ (ppm): 1.70 (s, 16H); 2.13 (m, 4H); 3.46
(t, 4H); 4.55 (t, 4H) ; 4.80 (t, 4H); 7.78 et 7.85 (d, 8H) ; 8.71 (s, 2H).
FT-IR, ν (cm⁻¹): 1692 (C=N); 1760 (NCO) ; 3350 (N, HCl).

### 1,12-bis[N,N'-(4-méthoxyphénylyloxycarbonyl)imidazolin-2-yl]dodécane: 6.9

A une suspension de 1 g (3.27 mmol) de 1,12-bis(imidazolin-2-yl)dodécane et de 0.89 ml (6.86 mmol) de triéthylamine dans 25 ml de chloroforme refroidie par un bain de glace, sont additionnés goutte à goutte et sous agitation 1.02 ml (6.86 mmol) de chloroformiate de 4-méthoxyphényle en solution dans 5 ml de chloroforme. Après quoi, l'agitation est maintenue à température ambiante toute une nuit. Le précipité isolé (chlorhydrate de triéthylamine) est par suite extrait plusieurs fois avec de l'éther. L'évaporation sous pression réduite de la phase éthérée, conduit à 1.31 g (66%) de produit sous forme d'huile.
RMN ¹H (CDCl₃, 100 MHz), δ (ppm): 1.21 (s, 16H); 1.63 (s, 4H); 2.71 (t, 4H); 3.75 (s, 8H); 3.88 (s 2H); 6.61 à 7.10 (m, 8H).
FT-IR, ν (cm⁻¹): 1177 et 1248 (C-O-C); 1646 (C=N); 1735 (NCO).
SM-ES+: [M+H]⁺: 607; [M+2H]²+/2 : 304 (100%)

### 1,12-bis[N,N'-(4-nitrophényloxycarbonyl)imidazolin-2-yl]dodécane: 6.10

A une suspension de 1 g (3.27 mmol) de 1,12-bis(imidazolin-2-yl)dodécane et de 0.89 ml (6.86 mmol) de triéthylamine dans 25 ml de chloroforme refroidie par un bain de glace, sont additionnés goutte à goutte et sous agitation 1.38 g (6.86 mmol) de chloroformiate de 4-nitrophényle en solution dans 5 ml de chloroforme. Après quoi, l'agitation est maintenue à température ambiante pendant 5 heures. Après évaporation sous pression réduite de la solution, le précipité formé est ensuite lavé à l'eau puis à l'acétone. Le solide jaune obtenu est par suite cristallisé par dissolution dans un minimum de dichlorométhane et par addition lente d'acétone. Le produit est isolé sous forme de cristaux blancs à 74% (1.54 g).
Point de fusion: 121-122°C
RMN ¹H (CDCl₃, 100 MHz), δ (ppm): 1.23 (s, 16H); 1.67 (m, 4H); 2.73 (t, 4H); 3.95 (s, 8H); 7.25 et 7.36 (dd, 4H);8.24 et 8.32 (dd, 4H).
FT-IR, ν (cm⁻¹): 1185 et 1196 (C-O-C); 1349 et 1524 (NO₂);1656 (C=N); 1748 (NCO).
SM-ES+: [M+H]⁺: 637; [M+2H]²+/2 : 319 (100%)

### 1,12-bis[N,N'-(acétoxyméthoxycarbonyl)imidazolin-2-yl] dodécane : 6.11

A une suspension de 1 g (3.27 mmol) de 1,12-bis(imidazolin-2-yl)dodécane et de 0.92 ml (6.54 mmol) de triéthylamine dans 20 ml de chloroforme refroidie par un bain de glace, sont additionnés goutte à goutte et sous agitation 1 g (6.54 mmol) de chloroformiate d'acétoxyméthyle en solution dans 5 ml de chloroforme. Après quoi, l'agitation est maintenue à température ambiante toute une nuit. Le mélange réactionnel est ensuite lavé successivement avec 20 ml d'eau, 20ml d'une solution aqueuse de bicarbonate de sodium à 5% puis 20 ml d'une solution aqueuse saturée en chlorure de sodium. Après séchage sur sulfate de sodium, la phase organique est évaporée sous pression réduite pour conduire à 2.95 g (84%) de produit sous forme d'une huile.
RMN ¹H (CDCl₃, 100 MHz), δ (ppm): 1.21 (s, 16H); 1.58 (m, 4H);2.08 (s, 6H); 2.65 (t, 4H); 3.74 (s, 8H); 5.74 (s, 4H).
FT-IR, ν (cm⁻¹): 1643 (C=N); 1682 (NCO); 1736 (OCO).
SM-ES+: [M+H]⁺: 539

### 1,12-bis[N,N'-((1-acétoxyéthoxy)carbonyl)imidazolin-2-yl]dodécane: 6.12

### a) Dichlorhydrate de 1,12-bis[N,N'-((1-chloroéthoxy) carbonyl)imidazolin-2-yl]dodécane

A une suspension de 2.50 g (8.17 mmol) de 1,12-bis(imidazolin-2-yl)dodécane et de 2.16 ml (16.75 mmol) de triéthylamine dans 40 ml de chloroforme refroidie par un bain de glace, sont additionnés goutte à goutte et sous agitation 1.8 ml (16.75 mmol) de chloroformiate de 1-chloroéthyle en solution dans 10 ml de chloroforme. Après quoi, l'agitation est maintenue à température ambiante toute une nuit. Le mélange réactionnel est ensuite filtré et le filtrat lavé successivement avec 100 ml d'eau, 100 ml d'une solution aqueuse saturée en chlorure de sodium et 3 x 100 ml d'eau. Après séchage sur sulfate de sodium, la phase organique est évaporée sous pression réduite. Le résidu refroidi est ensuite cristallisé à -4°C avec de l'hexane pour conduire à 3.91g (92%) de produit sous forme de poudre blanche.
RMN ¹H (CDCl₃, 100 MHz), δ (ppm): 1.19 (s, 16H); 1.58 (m, 4H); 1.77 (d, 6H); 2.64 (t, 4H); 3.74 (s, 8H); 6.49 (q, 2H).
FT-IR, ν (cm⁻¹):1087 (C-O-C); 1377 (CH₃ ; C-H) ; 1648 (C=N); 1737 (NCO).

### b) 1,12-bis[N,N'-((1-acétoxyéthoxy)carbonyl)imidazolin-2-yl]dodécane : 6.12

Une solution d'acide acétique contenant 3.20 g (6.17 mmol) de dichlorhydrate de 1,12-bis[*N*,*N*'-((1-chloroéthoxy)carbonyl)imidazolin-2-yl]dodécane et 5.9 g (18.50 mmol) d'acétate mercurique est laissée sous agitation et à température ambiante durant 72 heures. Après évaporation du solvant sous pression réduite, le résidu réactionnel est dilué dans 150 ml de chloroforme puis filtré. Le filtrat est ensuite lavé avec 3 x 300 ml d'une solution aqueuse saturée en chlorure de sodium. Le séchage sur sulfate de sodium et l'évaporée sous pression réduite de la phase organique conduit à 2.40 g (68%) de produit sous forme d'huile colorée.
RMN ¹H (DMSO-d₆, 100 MHz), δ (ppm): 1.24 (s, 16H); 1.44 (d, 6H) ; 1.52 (m, 4H); 2.03 (s, 6H) ; 2.61 (t, 4H); 3.79 (s, 8H); 6.69 (q, 2H).
FT-IR, ν (cm⁻¹) : 1072 et 1224 (C-O-C); 1645 (C=N); 1684 (NCO); 1733 (CO)
SM-ES+: [M+H]⁺: 567

### [1,12-bis(imidazolin-2-yl)dodécane]-1,12-bis-N,N'-phosphonate de diphényle: 6.13

A une suspension de 1 g (3.26 mmol) de 1,12-bis(imidazolin-2-yl)dodécane et de 0.96 ml (6.86 mmol) de triéthylamine dans 25 ml de chloroforme refroidie par un bain de glace, sont additionnés goutte à goutte et sous agitation 1.40 (6.70 mmol) de diphénylchlorophosphonate en solution dans 5 ml de chloroforme. Après quoi, l'agitation est maintenue à température ambiante toute une nuit. Le mélange réactionnel est ensuite filtré et le filtrat lavé successivement avec 100 ml d'eau, 100 ml d'une solution aqueuse saturée en chlorure de sodium et 2 x 100 ml d'eau. Après séchage sur sulfate de sodium, la phase organique est évaporée sous pression réduite pour conduire à 2.37 g (94%) de produit sous forme d'huile.
RMN ¹H (CDCl₃, 100 MHz), δ (ppm): 1.20 (s, 16H); 1.62 (m, 4H); 2.58 (t, 4H); 3.79 (s, 8H); 7.17 - 7.34 (m, 20H).
FT-IR, ν (cm⁻¹): 925 (P=O); 1160 et 1184 (C-O);1646 (C=N).

### [1,12-bis(imidazolin-2-yl)dodécane]-1,12-bis-N,N'-phosphonate de diéthyle: 6.14

A une suspension de 1.5 g (4.90 mmol) de 1,12-bis(imidazolin-2-yl)dodécane et de 1.5 ml (10.78 mmol) de triéthylamine dans 25 ml de chloroforme refroidie par un bain de glace, sont additionnés goutte à goutte et sous agitation 1.49 ml (10.29 mmol) de diéthylchlorophosphonate en solution dans 5 ml de chloroforme. Après quoi, l'agitation est maintenue à température ambiante toute une nuit. Le mélange réactionnel est ensuite filtré et le filtrat lavé successivement avec 100 ml d'eau, 100 ml d'une solution aqueuse saturée en chlorure de sodium et 2 x 100 ml d'eau. Après séchage sur sulfate de sodium, la phase organique est évaporée sous pression réduite pour conduire à 2.38 g (84%) de produit sous forme d'huile.
RMN ¹H (CDCl₃, 100 MHz), δ (ppm): 1.15 (s, 16H); 1.23 (t, 12H); 1.54 (m, 4H); 2.37 (t, 4H); 3.60 (t, 8H); 4.00 (quintuplet, 8H).
FT-IR, ν (cm⁻¹): 963 (P=O); 1016 et 1268 (C-O); 1640 (C=N).
SM-ES+: [M+H]⁺: 579; [M+H]²⁺/2 : 290 (100%)

### Dichlorhydrate de 1, 12-bis(1-méthyl imidazolin-2-yl)dodécane: 7.0, 2HCl

3.9 g (10 mmol) de dichlorhydrate de diéthyltétradécanediimidoate sont mis à reflux avec un excès de N-méthyl éthylènediamine pendant 24 heures. Après refroidissement de la solution, on ajoute de l'éther éthylique dans la solution. Les cristaux obtenus sont isolés par filtration, lavés à l'éther et séchés au dessiccateur, pour conduire à 2.9 g (71 %) de cristaux blancs.
Point de fusion: 166-170°C
RMN ¹H (DMSO-d₆, 100 MHz), δ (ppm): 1.38 (m, 16H); 1.67 (m, 4H); 2.47 (t, 4H); 2,90 (s, 6H); 3.35 (s, 8H); 9.28 (m, 2H).
FT-IR, ν (cm⁻¹): 1600 (C=N); 3031 (C=NH, HCl); 3200 (N-H)

### Dichlorhydrate de 1,12-bis(1,4,5,6-tétrahydropyrimidin-2-yl)dodécane: 8.0, 2HCl

A une solution de 1,16 g (3mmol) de dichlorhydrate de diéthyltétradécanediimidoate dans 15 ml d'éthanol absolu, sont ajoutés 0,51 ml (6,12 mmol) de 1,3-diaminopropane. Le mélange réactionnel est ensuite chauffé à reflux pendant 6 heures. Après addition de 4,5 ml d'eau, la température du mélange réactionnel est portée à 95°C pendant 4 heures. Après évaporation à sec, le résidu obtenu est cristallisé par addition d'éther puis agitation pendant 48 heures. Le précipité isolé par filtration, est ensuite lavé plusieurs fois par agitation forte dans l'éther et séché au dessiccateur, pour conduire à 0,83 g (68%) de cristaux blancs.
Point de fusion: 117-119°C
RMN ¹H (DMSO-d₆, 250 MHz), δ (ppm): 1,22 (s, 16H); 1,55 (m, 4H); 1,80 (m, 4H); 2,45 (t, 4H); 3,25 (s, 8H); 9.28 (m, 2H).

### Dichlorhydrate de 1,12-bis(4-méthyl imidazolin-2-yl)dodécane: 9.0, 2HCl

A une solution de 0,96 g (2,5 mmol) de dichlorhydrate de diéthyl tétradécanediimidoate dans 12 ml d'éthanol anhydre, sont ajoutés sous agitation, 0,44 ml (5,1 mmol) de 1,2-diaminopropane. Le mélange est ensuite chauffé à une température de 90°C durant 48 heures. Après évaporation du solvant, le résidu obtenu est solubilisé dans de l'éthanol puis filtré. Le filtrat est évaporé puis de l'éther est additionné et éliminé sous pression réduite afin d'entraîner les restes de solvant. 0,71 g (70%) de produit sont alors obtenu sous forme de poudre blanche.
RMN ¹H (DMSO-d₆, 250 MHz), δ (ppm): 1,30 (m, 22H); 1,64 (m, 4H); 2,52
(t, 4H); 3,40 (t, 2H); 3,95 (t, 2H); 3,95 (m, 2H); 9,32 (m, 2H).

### Dichlorhydrate de 1,12-bis(4,4-diméthyl imidazolin-2-yl)dodécane: 10.0, 2HCl

Le mélange réactionnel composé de 0,96 g (2,5 mmol) de dichlorhydrate de diéthyl tétradécanediimidoate dans 12 ml d'éthanol anhydre et de 0,54 ml (5,1 mmol) de 1,2-diamino-2-méthylpropane est chauffé à 90°C pendant 48 heures. Le solvant est évaporé, et le résidu est solubilisé dans de l'éthanol à chaud. Après refroidissement à température ambiante, le solide formé est filtré puis traité à l'éther comme le composé **9.0,** 2HCl pour conduire à 0,76 g (70%) de produit sous forme de poudre beige.
RMN ¹H (DMSO-d₆, 250 MHz), δ (ppm): 1,24 (s, 12H); 1,32 (s, 16H); 1,62 (m, 4H); 2,50 (t, 4H); 3,54 (s, 4H); 9,30 (m, 2H).

### Dichlorhydrate de 1,12-di(3a,4,5,6,7,7a-hexahydro-1H-benzimidazo-2-yl)dodécane: 11.0, 2HCl

0,96 g (2,5 mmol) de dichlorhydrate de diéthyltétradécanediimidoate dans 12 ml d'éthanol anhydre et 0,63 ml de 1,2-diaminocyclohexane sont mis à reflux (90°C) pendant 48 heures. Après évaporation du mélange, on rajoute de l'éthanol dans la solution, on filtre et le filtrat évaporé est traité comme décrit pour le composé 9.0, 2HCl. On obtient 0,82 g (67%) de cristaux.
RMN ¹H (DMSO-d₆, 250 MHz), δ (ppm): 1,25 (s, 16H); 1,30-1,65 (m, 16H); 1,70 (m, 4H); 2,50 (t, 4H); 3,34 (m, 2H); 4,10 (m, 2H); 9,32 (m, 2H).

### 1,12-bis[(1,2,4-oxadiazole)-3-yl]dodécane: 18.0

A une suspension sous agitation de 2 g (7 mmol) de bis-amidoxime 1.15 dans 23.26 ml (139.86 mmol) d'orthoformiate d'éthyle, sont ajoutés 0.6 ml (4.9 mmol) de diéthyltrifluoroborane. Le mélange réactionnel est laissé sous agitation à température ambiante durant 15 minutes puis chauffé à reflux pendant 1 heure. A la solution obtenue sont ajoutés 150 ml d'acétate d'éthyle, et le mélange est lavé successivement avec de l'eau (100 ml), une solution saturée de bicarbonate de sodium (100 ml) et avec une solution saturée de chlorure de sodium (100 ml). La phase organique est ensuite séchée sur sulfate de sodium et évaporée sous pression réduite. Le résidu obtenu est lavé avec de l'éther froid puis séché pour conduire à 1.50 g (70 %) de produit sous forme de poudre blanche.
Point de fusion: 92-93 °C.
RMN ¹H (CDCl₃, 100 MHz), δ (ppm): 1.24 (s, 16H); 1.73 (m, 4H); 2.77 (t, 4H); 8.61 (s, 2H).
FT-IR, ν (cm⁻¹): 1111 (C-O); 1558 (C=N); 3123 (=CH).

### 1,12-bis[(5-méthyl-1,2,4-oxadiazole)-3-yl]dodécane: 18.1

Une suspension sous agitation de 1.5 g (4.05 mmol) de 1.22 dans 30 ml de xylène, est chauffée à 150°C durant 2 heures. Le mélange réactionnel est ensuite évaporé sous pression réduite. Le résidu solide obtenu est par suite recristallisé avec de l'éther de pétrole pour conduire après séchage au dessiccateur, à 1.10 g (81%) de produit sous forme de poudre blanche.
Point de fusion: 63-64 °C.
RMN ¹H (CDCl₃, 100 MHz), δ (ppm): 1.23 (s, 16H); 1.69 (m, 4H); 2.53 (s, 6H); 2.66 (t, 4H).
FT-IR, ν (cm⁻¹): 1581 (C-N); 1640 (C=N)

### 1,12-bis[(5-trifluorométhyl-1,2,4-oxadiazole)-3-yl]dodécane: 18.4

Dans 20 ml (139.86 mmol) d'anhydride trifluoroacétique sous agitation et refroidie par un bain d'eau glacée, sont ajoutés portion par portion 1 g (3.50 mmol) de bis-amidoxime **1.15.** L'agitation est maintenue durant 30 minutes à froid jusqu'à dissolution complète de **1.15.** Au mélange réactionnel, sont ajoutés 50 ml d'éther et très lentement, 100 ml d'eau froide (réaction exothermique). La phase éthérée est ensuite lavée successivement avec 2 X 100 ml d'eau, 2 X 50 ml d'une solution de soude 1N et 100 ml d'eau. La phase organique est par suite séchée sur sulfate de sodium et évaporée sous pression réduite. Après refroidissement, le résidu cristallise pour donner 1.39 g (90%) de produit sous forme de cristaux blancs.
Point de fusion: < 40 °C.
RMN ¹H (CDCl₃, 100 MHz), δ (ppm): 1.26 (s, 16H); 1.75 (m, 4H); 2.81 (t, 4H).
FT-IR, ν (cm⁻¹): 766 et 1150 (CF₃); 1519 (C-N); 1608 (C=N)

### 1,12-bis[(5-trichlorométhyl-1,2,4-oxadiazole)-3-yl]dodécane: 18.5

Une suspension sous agitation de 3 g (10.49 mmol) bis-amidoxime 1.15 et de 13.71 g (83.92 mmol) d'acide trichloracétique dans 10 ml de chloroforme est chauffée à 85°C jusqu'à obtention d'une solution. A la solution, est ajouté en trois portions égales, 4.70 ml (41.96 mmol) de trichloroacetyl chlorure. Le mélange réactionnel est ensuite chauffé à 94°C durant 45 minutes. Après refroidissement, 200 ml d'acétate d'éthyle sont ajoutés puis le mélange est lavé successivement avec une solution saturée de carbonate de sodium (2 X 100 ml), une solution saturée de chlorure de sodium (2 X 100 ml) et avec 100 ml d'eau. La phase organique est ensuite séchée sur sulfate de sodium et évaporée sous pression réduite. Le résidu obtenu est séché pour conduire à 5.19 g (91 %) de produit sous forme de poudre jaune.
Point de fusion: 55-56 °C.
RMN ¹H (CDCl₃, 100 MHz), δ (ppm): 1.26 (s, 16H); 1.76 (t, 4H); 2.78 (t, 4H).
FT-IR, ν (cm⁻¹) : 798 et 830 (CCl₃); 1573 (C=N).

### 1,12-bis[(5-phényl-1,2,4-oxadiazole)-3-yl]dodécane: 18.6

Une suspension sous agitation de 1.5 g (3.04 mmol) de 1.23 dans 30 ml de xylène, est chauffée à 150°C durant 2 heures. La solution réactionnelle est ensuite décantée puis évaporée sous pression réduite. Le résidu solide obtenu est par suite recristallisé avec de l'éther de pétrole pour conduire après séchage au dessiccateur, à 1.12 g (80%) de produit sous forme de poudre colorée
Point de fusion: 97-98 °C.
RMN ¹H (CDCl₃, 100 MHz), δ (ppm): 1.25 (s, 16H); 1.78 (m, 4H); 2.77 (t, 4H); 7.48 - 7.54 (m, 6H); 8.06 - 8.14 (m, 4H).
FT-IR, ν (cm⁻¹) : 1581 (C-N); 1640 (C=N)

### 1,12-bis[(5-éthyloxycarbonyl-1,2,4-oxadiazole)-3-yl]dodécane: 18.7

A une suspension sous agitation de 2 g (7 mmol) bis-amidoxime 1.15, de 4 ml (49.50 mmol) de pyridine et de 4 g de tamis moléculaire 4A dans 50 ml de chloroforme, sont ajoutés 2.35 ml (21 mmol) de chlorure d'oxalyl éthyle. Le mélange réactionnel est ensuite chauffé à 80°C durant 16 heures puis filtré et évaporé sous pression réduite Au résidu, 100 ml d'acétate d'éthyle sont ajoutés puis la solution est lavée successivement avec une solution saturée de carbonate de sodium (2 X 50 ml), une solution saturée de chlorure de sodium (2 X 50 ml) et avec 50 ml d'eau. La phase organique est ensuite séchée sur sulfate de sodium et évaporée sous pression réduite. Le résidu froid obtenu est lavé avec de l'éthanol froid puis séché pour conduire à 1.96 g (62%) de produit sous forme de poudre.
Point de fusion: 70-71 °C.
RMN ¹H (CDCl₃, 100 MHz), δ (ppm): 1.24 (s, 16H); 1.44 (t, 6H) 1.77 (m, 4H); 2.80 (t, 4H); 4.51 (quartet, 4H).
FT-IR, ν (cm⁻¹) : 1029 et 1197 (C-O-C); 1584 (C=N); 1760 (COO)

### 1,12-bis[(5-carbamoyl-1,2,4-oxadiazole)-3-yl]dodécane: 18.9

A une solution d'éthanol ammoniacal 10% (90 ml) sont ajoutés 2 g (4.44 mmol) de 18.7. Le mélange réactionnel fermé hermétiquement est laissé sous agitation et à température ambiante durant 24 heures. La suspension est ensuite filtrée et lavée avec de l'éthanol froid puis séchée. 1.58 g (91%) de produit sont ainsi isolés sous forme de poudre jaune.
Point de fusion: 196-197 °C.
RMN ¹H (DMSO-d₆, 100 MHz), δ (ppm): 1.71 (s, 16H); 2.14 (m, 4H); 3.23 (t, 4H); 8.98 (s, 2H).
FT-IR, ν (cm⁻¹) : 1573 (C-N); 1604 (C=N); 1673 (CON); 3233 et 3432 (NH₂)

### 1,12-bis[(5-cyano-1,2,4-oxadiazole)-3-yl]dodécane: 18.10

A une suspension refroidie à 0°C de 1.5 g (3.83 mmol) de 18.9 et de 1.55 ml (19.13 mmol) de pyridine dans 60 ml de dioxane, sont ajoutés 1.3 ml (9.18 mmol) d'anhydride trifluoroacétique. Le mélange réactionnel est laissé sous agitation à température ambiante durant 16 heures. La solution obtenue est ensuite diluée avec 150 ml d'acétate d'éthyle puis lavée successivement avec de l'eau (100 ml) et avec une solution saturée de chlorure de sodium (2 X 100 ml). La phase organique séchée sur sulfate de sodium et évaporée sous pression réduite conduit à 0.83 g (61 %) de produit sous forme d'huile colorée.
RMN ¹H (CDCl₃, 100 MHz), δ (ppm): 1.24 (s, 16H); 1.73 (m, 4H); 2.81 (t, 4H).
FT-IR, ν (cm⁻¹) : 1561 (C=N); 2260 (CN)

### 1,12-bis(N,N'-dibenzyloxycarbonylguanidino)dodécane: 12.1

Une solution de 1 g (5mmol) de 1,12-diaminododécane et de 3.76 g (10.5mmol) de *N,N'*-di-benzyloxycarbonyl-S-méthylisothiourée dans 70 ml de tétrahydrofurane est chauffée entre 60 et 70°C pendant 24 H. Après quoi, le solvant est évaporé sous pression réduite. Le résidu obtenu est ensuite repris avec du dichlorométhane et lavé successivement avec une solution aqueuse de bicarbonate de sodium à 5%, une solution aqueuse saturée de chlorure de sodium et d'eau. La phase organique est par suite, séchée sur du sulfate de sodium, évaporée sous pression réduite, et purifiée par chromatographie sur colonne de silice (DCM). Les différentes fractions réunies et évaporées sous pression réduite conduisent à une huile jaune qui cristallise dans l'éther pour donner 2.4 g (58%) de produit sous forme de poudre blanche.
Point de fusion: 97- 98 °C
RMN ¹H (CDCl₃, 100 MHz), δ (ppm): 1.24 (s, 16H); 1.54 (s, 4H); 3.40 (q, 4H); 5.11 (s, 4H); 5.16 (s, 4H); 7.15- 7.54 (m, 20H); 8.28 (t, 2H); 11.73 (s, 2H)
FT-IR, ν (cm⁻¹): 1054 et 1130 (C-O); 1651 (C=N); 1736 (NCO); 3130 (NHCO); 3333 (NH)
SM-FAB⁺: [M+H]⁺: 821; [M+2H]⁺⁺/2: 412

### 1,12-bis(N,N'-di-tert-butyloxycarbonylguanidino)dodécane : 12.2

Un mélange de 1 g (5 mmol) de 1,12-diaminododecane et de 3.19 g (11 mmol) de *N,N'*-di-*tert*-butyloxycarbonyl-S-méthylisothiourée dans 100 ml de méthanol, est chauffé entre 50 et 60°C durant 48 heures. Après évaporation sous pression réduite de la solution, le résidu obtenu est repris avec 100 ml de DCM et lavé successivement avec une solution aqueuse de NaHCO₃ à 5% (2 X 100 ml), de l'eau (2 X 100 ml) et avec 100 ml d'une solution saturée de NaCl. La phase organique est ensuite séchée sur sulfate de sodium, évaporée sous pression réduite et purifiée sur silice (DCM/MeOH 98%) pour conduire à 2.50 g (73%) de produit se cristallisant dans de l'éther de pétrole à froid sous forme de poudre.
Point de fusion: 114- 116 °C
RMN ¹H (CDCl₃, 100 MHz), δ (ppm): 1.24 (s, 16H); 1.47 (s, 36H); 1.61 (m, 4H)3.35 (q, 4H); 8.27 (t, 2H); 11.48 (s, 2H)
FT-IR, ν (cm⁻¹) : 1028 et 1138 (C-O); 1670 (C=N); 1740 (NCO); 3132 (NHCO); 3314 (NH)
SM-ES+: [M+H]⁺: 685

### Diiodhydrate de 1,12-bis[N,N'-(2-amino-3,4,5,6-tétrahydropyrimidyl)]dodécane: 16.0, 2HI

Le mélange réactionnel composé de 0,72 g (3,6 mmol) de 1,12-dodécanediamine, de 1,86 g (7,2 mmol) d'iodure de 2-méthylsulfanyl-3,4,5,6-tétrahydropyrimidinium et de 0,5 ml (3,6 mmol) de triéthylamine dans 20 ml d'acétonitrile est chauffé à reflux pendant 22h.. Après refroidissement à température ambiante, le solvant de réaction est évaporé et le résidu est chromatographié sur colonne de silice (CH₂Cl₂/CH₃OH/NH₄OH 89:10:1). 0,91 g de sel de guanidinium sont obtenus, soit un rendement de 41 %.
RMN ¹H (CD₃OD, 100 MHz), δ (ppm) : 1,49 (m, 20H) ; 2,13 (m, 4H) ; 3,33 (m, 4H) ; 3,56 (m, 8H).
SM-FAB⁺: [M+H]⁺: 365; [M+H+HI]⁺: 493

### Diiodhydrate de 1,12-bis[N,N'-(5,5-diméthyl-3,4,5,6-tétrahydropyrimidin-2-yl)amino] dodécane: 17.0, 2HI

Le mélange réactionnel composé de 0,72 g (3,6 mmol) de 1,12-dodécanediamine, de 2,06 g (7,2 mmol) d'iodhydrate 5,5-diméthyl-2-méthylsulfanyl-3,4,5,6-tétrahydropyrimidinium et de 0,5 ml (3,6 mmol) de triéthylamine dans 20 ml d'acétonitrile est chauffé à reflux pendant 22h. Après refroidissement à température ambiante, le solvant de réaction est évaporé et le résidu est chromatographié sur colonne de silice (CH₂Cl₂/CH₃OH/NH₄OH 89:10:1). 0,91 g de sel sont obtenus, soit un rendement de 36%.
RMN ¹H (CD₃OD, 100 MHz), δ (ppm): 1,17 (s, 12H); 1,49 (m, 20H); 3,07
(s, 8H); 3,23 (m, 4H).

### Ditrifluoroacétate de [1-[N-(5,5-diméthyl-3,4,5,6-tétrahydropyrimidin-2-yl)amino]-12-[N'-(3,4-dihydro-2H-pyrrol-5-yl)amino]]dodécane: 25.0,2TFA

Le mélange réactionnel composé de 1 mmol de ditrifluoroacétate de 1-[N-(5,5-diméthyl-3,4,5,6-tétrahydropyrimidinium-2-yl)amino]dodécane-12-ammonium, 1mmol (léq.) de 3,4-dihydro-5-méthoxy-2*H*-pyrrole et 0,5 ml de triéthylamine dans 10 ml d'éthanol absolu est chauffé à reflux pendant 20 h.. Après refroidissement à température ambiante, le mélange réactionnel est évaporé à sec et chromatographié sur colonne de silice (CH₂Cl₂/CH₃OH/NH₄OH 85:13:2) pour donner le produit, sous forme d'huile, avec un rendement de 65%.
RMN ¹H (CD₃OD, 360 MHz), δ (ppm) : 1,12 (s, 6H) ; 1,36 (m, 16H) ; 1,60 (m, 2H) ; 1,69 (m, 2H) ; 2,28 (m, 2H) ; 2,93 (m, 2H) ; 3,09 (s, 4H) ; 3,22 (m, 2H) ; 3,31 (m, 2H) ; 3,75 (m, 2H).
SM-ES⁺: [M+H]⁺: 378 ; [M+H+TFA]⁺: 492 ; [M+2H]⁺⁺/2 : 189,5

### 1,12-bis[N,N'-(3,4-dihydro-2H-pyrrol-5-yl)amino]dodécane : 21.0

A une solution de 2.76 g (10.1 mmol) de chlorhydrate de 1,12-diaminododécane dans 70 ml d'éthanol absolu, sont ajoutés 2.5 g (25,25 mmol) de 2-méthoxypyrroline. Le mélange réactionnel est laissé sous agitation et à température ambiante pendant 24 H. La solution est par suite évaporée sous pression réduite et le résidu après refroidissement, est repris avec 100 ml d'eau puis alcalinisé avec une solution de soude 0.1 N. Après quoi, le précipité formé est essoré, lavé à l'eau puis à l'éther et séché au dessiccateur. On obtient 3,27 g (97%) du produit attendu sous forme de poudre blanche.
Point de fusion: 155-156°C
RMN ¹H (CDCl₃, 100 MHz), δ (ppm): 1.23 (s, 16H); 1.49 (m, 4H); 1.89 (quint., 4H); 2.38 (t, 4H); 3.21 (t, 4H); 3.43 (s, 2H); 3.62 (t, 4H)
FT-IR, ν (cm⁻¹): 1630 (C=N); 3049 et 3221 (NH)

### 1,12-bis(N,N'-acetamidinyl)dodécane: 20.0

Un mélange de 2 g (10 mmol) de 1,12-diaminododécane et de 2,47 g

(20 mmol) de chlorhydrate d'éthylacétimidate dans 25 ml de dioxane anhydre est chauffé à reflux durant 24 heures. Après refroidissement à température ambiante, on ajoute une solution aqueuse de KOH 1N qui entraîne la formation d'un précipité. Ce précipité est lavé au méthanol à chaud puis filtré. Après passage au dessiccateur plusieurs heures, 2,06 g (7,3 mmol) de produit sont obtenus, soit un rendement de 73%.
Point de fusion: 92-97°C
RMN¹H (DMSOd₆, 250 MHz), δ (ppm) : 1,3 (m, 16H) ; 1,5 (m, 4H) ; 1,8 (s, 6H); 2,9 (t, 4H); 5,5 (m, 2H)

### 1,12-bis (N,N'-hydroxyacétamidinyl) dodécane, 20.1 et son dichlorhydrate, 20.1, 2HCl.

A une solution de 12.9 g (125 mmol) *N*-hydroxyacétimidate d'éthyle dans 125 ml d'éthanol, sont ajoutés 10 g (50 mmol) de 1,12-diaminododécane. Le mélange réactionnel est chauffé à 80°C durant 4 jours. Après refroidissement, le précipité formé est essoré puis lavé plusieurs fois à l'éthanol et à l'éther, séché au dessiccateur et recristallisé dans l'éthanol, pour conduire à 7.26 g (46 %) d'hydroxyacétamidinyle 20.1 sous forme de poudre blanche (Point de fusion: 150-151°C).

1 g d'hydroxyacétamidinyle 20.1 est dissous dans 20 ml d'une solution d'éthanol anhydre saturée en acide chlorhydrique gaz. Le mélange sous agitation vive est chauffé à 50°C durant 2H. A la solution froide sont additionnés 100 ml d'éther anhydre et le mélange est laissé au repos. Le solide formé est essoré, séché, dissous dans 100 ml d'eau distillée puis filtrée. Le filtrat aqueux est lyophilisé pour conduire au produit sous forme de poudre blanche.
RMN ¹H (DMSO-d₆, 100 MHz): 1.72 (s, 16H, H₃-H₁₀), 1.96 (s, 4H, H₂ et H₁₁); 2.62 (s, 6H, 2H_{2'}); 3.68 (q, 4H, H₁ et H₁₂), 9.30 (t, 2H, 2NH); 11.40 (s, 2H, 2NOH); 12.92 (s, 2H, 2C=NOH,HCl).
FT-IR: 1683 (C=N); 3002; 3142 et 3202 (NH; C=NOH,HCl et NOH).

### 1,12-bis (N,N'-méthoxyacétamidinyl) dodécane, 20.2.

A une solution hydro-alcoolique de soude [préparée à partir de 0.45 g (11.15 mmol) de soude et de 25 ml d'éthanol/eau (4:1)] est ajouté 1 g (3.18 mmol) d'hydroxyacétamidinyle 20.1. Après 30 minutes d'agitation, est additionné goutte à goutte à la suspension réactionnelle, 0.42 ml (6.68 mmol) d'iodure de méthyle. Le mélange est agité à température ambiante durant 24H. La solution trouble est ensuite filtrée et le filtrat évaporé sous pression réduite. Le résidu obtenu est repris avec 50 ml de chloroforme puis lavé avec une solution aqueuse saturée en NaCl (2 x 100 ml). La phase organique est séchée sur sulfate de sodium et évaporée sous pression réduite pour conduire à 1.05 g (96%) de méthoxyacétamidinyle sous forme huile colorée.
RMN ¹H (CDCl₃, 100 MHz): 1.24 (s, 16H, H₃-H₁₀), 1.45 (m, 4H, H₂ et H₁₁); 1.83 (s, 6H, 2H_{2'}); 3.05 (q, 4H, H₁ et H₁₂), 3.70 (s, 6H, 2H_{3'}); 5.03 (t, 2H, 2NH).
ES⁺ SM: 343 [M+H⁺]; 172 (100%) [(M+2H⁺)/2].
FT-IR: 1637 (C=N); 3264 (NH).

### 1,12-bis (N,N'-acétoxyacétamidinyl) dodécane: 20.8

Dans 12 ml (127.4 mmol) d'anhydride acétique sous agitation et refroidie à 0°C, est ajouté par petites portions 1 g (3.18 mmol) d'hydroxyacétamidinyle **20.1.** L'agitation est maintenue durant 2H à température ambiante. Au mélange réactionnel, sont ajoutés 100 ml de chloroforme. La solution est lavée successivement avec 2 x 100 ml d'une solution aqueuse saturée de chlorure de sodium, 3 x 100 ml d'une solution de soude 2N et 2 x 100 ml d'eau. La phase organique est par suite séchée sur sulfate de sodium et évaporée sous pression réduite. 30 ml d'éther sont ajoutés au résidu huileux froid, puis la solution est laissée au réfrigérateur durant 16H. Le solide formé est ensuite trituré dans l'éther puis essoré. La poudre obtenue est finalement lavée avec de l'éther et séchée pour conduire à 1.12 g (89%) d'acétoxyacétamidinyle sous forme de poudre blanche. Point de fusion: 68-69°C.
RMN ¹H (CDCl₃, 100 MHz): 1.24 (s, 16H, H₃-H₁₀), 1.44 (m, 4H, H₂ et H₁₁); 1.92 (s, 6H, 2H_{2'}); 2.11 (s, 6H, 2H_{4'}); 3.10 (q, 4H, H₁ et H₁₂); 5.04 (t, 2H, 2NH).
ES⁺ SM: 399 (100%) [M+H⁺]; 200 [(M+2H⁺)/2.
FT-IR: 1623 (C=N); 1742 (OCO); 3333 (NH).

### 1,12-bis (N,N'-benzoyloxyacétamidinyl) dodécane, 20.9.

A une suspension sous agitation et refroidie à 0°C de 1 g (3.18 mmol) d'hydroxyacétamidinyle **20.1** et de 0.94 (6.68 mmol) ml de triéthylamine dans 30 ml de chloroforme, est ajouté goutte à goutte 0.78 ml (6.68 mmol) de chlorure de benzoyle dans 5 ml de chloroforme. L'agitation est maintenue durant 3H à température ambiante. Le mélange réactionnel est ensuite lavé avec 2 x 100 ml d'eau puis avec 100 ml d'une solution saturée en NaCl. La phase organique est par suite séchée sur sulfate de sodium et évaporée sous pression réduite. Le résidu huileux est laissé au repos toute une nuit au réfrigérateur. Le solide formé est trituré dans de l'éther froid, lavé avec de l'éther puis essoré et séché pour conduire à 1.27 g (77%) de benzoyloxyacétamidinyle sous forme de poudre blanche. Point de fusion: 95-96°C.
RMN ¹H (CDCl₃, 100 MHz): 1.23 (s, 16H, H₃-H₁₀), 1.51 (m, 4H, H₂ et H₁₁); 2.02 (s, 6H, 2H_{2'}); 3.15 (q, 4H, H₁ et H₁₂), 5.19 (t, 2H, 2NH); 7.34-7.54 [m, 6H, 2(H_{6'}-H_{8'})];.7.93-8.03 [dd, 4H, 2(H_{5'} et H_{9'})].
FT-IR:1279 (C-O-C); 1622 (C=N); 1714 (OCO); 3385 (NH).
ES⁺ SM: 523 (100%) [M+H⁺]; 262 [(M+2H⁺)/2.

### 1,12-bis (N,N'-éthylcarbamoyloxyacétamidinyl) dodécane: 20.10

A une suspension sous agitation de 1 g (3.18 mmol) d'hydroxyacétamidinyle 20.1 et de 0.45 g (3.18 mmol) de carbonate de potassium dans 40 ml de chloroforme, est ajouté goutte à goutte 0.53 ml (6.69 mmol) d'éthylisocyanate. L'agitation est maintenue toute une nuit à température ambiante. Le mélange réactionnel est filtré et le filtrat lavé avec 3 x 100 ml d'eau .La phase organique est par suite séchée sur sulfate de sodium et évaporée sous pression réduite pour conduire à 1.32 g (91%) d'éthylcarbamoyloxyacétamidinyle sous forme d'huile colorée.
RMN ¹H (CDCl₃, 100 MHz): 1.14 (t, 6H, 2H_{5'}); 1.22 (s, 16H, H₃-H₁₀); 1.44 (m, 4H, H₂ et H₁₁); 1.84 (s, 6H, 2H_{2'}); 3.07 (q, 4H, H₁ et H₁₂); 3.26 (quintuplet, 4H, 2H_{4'}); 5.33 (t, 2H, 2NH); 6.49 (t, 2H, 2NHCO).
FT-IR: 1215 (C-O); 1641 (C=N); 1705 (OCON); 3345 (NHCO); 3398 (NH).

### 1,12-bis (N,N'-phénylcarbamoyloxyacétamidinyl) dodécane: 20.11

A une suspension sous agitation de 1 g (3.18 mmol) d'hydroxyacétamidinyle 20.1 et de 0.45 g (3.18 mmol) de carbonate de potassium dans 40 ml de chloroforme, est ajouté goutte à goutte 0.73 ml (6.69 mmol) de phénylisocyanate. L'agitation est maintenue toute une nuit à température ambiante. Le mélange réactionnel est ensuite filtré et le filtrat lavé avec 3 x 100 ml d'eau .La phase organique est par suite séchée sur sulfate de sodium et évaporée sous pression réduite. Le résidu solide obtenu est lavé plusieurs fois avec de l'éther puis séché au dessiccateur pour conduire à 1.53 g (87%) de phénylcarbamoyloxyacétamidinyle sous forme de poudre blanche. Point de fusion: 103-104°C
RMN ¹H (CDCl₃, 100 MHz): 1.26 (s, 16H, H₃-H₁₀); 1.51 (m, 4H, H₂ et H₁₁); 1.94 (s, 6H, 2H_{2'}); 3.13 (q, 4H, H₁ et H₁₂); 5.46 (t, 2H, 2NH); 7.0-7.52 (m, 10H, H aromatique.); 8.61 (s, 2H, 2NHCO).
FT-IR: 1663 (C=N); 1717 (OCON); 3291 (NHCO); 3443 (NH).
ES⁺ SM: 457 (100%) [M+H⁺]; 229 [(M+2H⁺)/2.

### 1,12-bis (N,N'-méthylsulfonyloxyacétamidinyl) dodécane: 20.12

A une suspension sous agitation et refroidie entre 0 et 5°C par un bain de glace de 1 g (3.18 mmol) d'hydroxyacétamidinyle **20.1** et de 0.55 ml (6.68 mmol) de pyridine dans 30 ml de chloroforme, est ajouté goutte à goutte 0.52 ml (6.68 mmol) de chlorure de méthylsulfonyle dans 5 ml de chloroforme. L'agitation est maintenue durant 4H entre 10 et 15°C. Le mélange réactionnel est ensuite lavé avec 3 x 100 ml d'eau. La phase organique est séchée sur sulfate de sodium et évaporée sous pression réduite. Le résidu huileux est laissé une nuit au réfrigérateur. Le solide formé est trituré dans de l'éther froid, lavé avec de l'éther puis essoré et séché pour conduire à 1.33 (89%) g de méthylsulfonyloxyacétamidinyle sous forme de poudre blanche. Point de fusion: 67-68°C.
RMN ¹H (CDCl₃, 100 MHz): 1.25 (s, 16H, H₃-H₁₀); 1.50 (m, 4H, H₂ et H₁₁); 1.91 (s, 6H, 2H_{2'}); 3.11 (s, 6H, 2H_{3'}); 3.15 (q, 4H, H₁ et H₁₂); 5.23 (t, 2H, 2NH).
FT-IR: 1637 (C=N); 3302 (NH).
ES⁺ SM: 471 (100%) [M+H⁺].

### [1,12-bis (acétamidinyl) dodécane]-1,12-bis-N,N'-phosphate de diéthyle, 20.13.

A une suspension sous agitation et refroidie entre 0 et 5°C de 1 g (3.18 mmol) d'hydroxyacétamidinyle 20.1 et de 0.94 ml (6.68 mmol) de triéthylamine dans 30 ml de DMF, est ajouté goutte à goutte 0.97 ml (6.68 mmol) de diéthylchlorophosphonate dans 5 ml de chloroforme. L'agitation est maintenue toute une nuit. Le mélange réactionnel est filtré et le filtrat repris avec 100 ml d'acétate d'éthyle. La phase organique est lavée avec 3 x 100 ml d'eau puis séchée sur sulfate de sodium et évaporée sous pression réduite. Le résidu est repris avec 100 ml chloroforme puis à nouveau lavé avec 3 x 100 ml d'eau, séché sur sulfate de sodium et évaporée sous pression réduite pour conduire à 1.13 g (61 %) de 20.13 sous forme d'huile colorée.
RMN ¹H (CDCl₃, 100 MHz): 1.25 (s, 16H, H₃-H₁₀); 1.33 (t, 12H, 4H_{4'}); 1.43 (m, 4H, H₂ et H₁₁); 1.87 (s, 6H, 2H_{2'}); 3.08 (q, 4H, H₁ et H₁₂); 4.18 (quint, 8H, 4H_{3'}); 5.28 (t, 2H, 2NH).
FT-IR: 1634 (C=N); 3316 (NH).

### 1,12-bis [N,N'(3-méthyl-1,2,4-oxadiazol-5(4H)-one)-3-yl] dodécane: 20.14

A une suspension sous agitation de 1 g (3.18 mmol) d'hydroxyacétamidinyle 20.1 et de 0.45 g (3.18 mmol) de carbonate de potassium dans 30 ml de chloroforme, est ajouté goutte à goutte 0.50 ml (6.68 mmol) de méthylchloroformiate dans 5 ml de chloroforme. L'agitation est maintenue durant 1H à température ambiante puis la suspension est chauffée autour de 50°C durant 45 minutes. Le mélange réactionnel froid est ensuite filtré, lavé avec 3 x 100 ml d'eau. La phase organique est par suite séchée sur sulfate de sodium et évaporée sous pression réduite. Le résidu est laissé une nuit au réfrigérateur. Le solide formé est finalement trituré dans de l'éther froid, lavé avec de l'éther puis essoré et séché pour conduire 0.92 g (79%) d'oxadiazolone sous forme de poudre blanche. Point de fusion: 53-54°C.
RMN ¹H (CDCl₃, 00 MHz): 1.24 (s, 16H, H₃-H₁₀); 1.64 (s, 4H, H₂ et H₁₁); 2.24 (s, 6H, 2H_{2'}); 3.52 (t, 4H, H₁ et H₁₂).
FT-IR: 1599 (C=N); 1754 (OCO).

### 1,12-bis [N,N' 2-hydroxyacetamidinyl)] dodécane, 26.0.

### a) Hydroxyacetonitrile ou Glycolonitrile

A une solution aqueuse (60 ml) refroidie à 0°C de 15 g (306 mmol) de cyanure ce sodium, sont ajoutés goutte à goutte 22.73 g (303 mmol) d'une solution de formaldehyde (40% dans l'eau). L'agitation est maintenue durant 30 mn à cette température. Le pH de la solution est successivement ajusté à 2 avec du H₂SO₄ 7.5N (45 ml) et à 5 avec du Na₂CO₃. Le sulfate de sodium éventuellement formé est dissous par ajout d'eau et la solution est ensuite extraite à l'éther (4 x 200 ml). La phase organique est séchée sur sulfate de sodium anhydre et évaporée sous pression réduite pour donner 13.13 g (76%) l'hydroxyacetonitrile sous forme d'huile colorée. Cette huile, non purifiée, est utilisée directement. Elle se décompose à température ambiante au bout de 24H.
RMN ¹H (CDCl₃, 100 MHz): 2.30 (s, 1H, OH); 4.34 (s, 2H, CH₂).
FT-IR: 2259 (C=N); 3425 (OH).

### b) Chlorhydrate d'ethyl hydroxyacetimidate

De l'HCl gaz est barboté dans une solution refroidie à 0°C de 20 ml d'éthanol anhydre, 40 ml d'éther anhydre et 9 g (158 mmol) d'hydroxyacetonitrile, durant 1H30. Le mélange réactionnel est laissé agitater une nuit à température ambiante. Le précipité forme est ensuite essoré et lavé plusieurs fois à l'éther puis séché au dessiccateur pour conduire à.19.52 g (89%) de chlorhydrate hydroxyacétimidate d'éthyle sous forme de poudre blanche.
RMN ¹H (DMSO-d₆, 100 MHz): 1.80 (t, 3H, CH₃); 4.80 (s, 2H, CH₂); 4.85 (q, 2H, CH₂); 11.73 (sl, 2H, NH,HCl).
FT-IR: 1645 (C=N); 3119 (OH); 3229 (NH,HCl).

### 1,12-bis (N,N' 2-hydroxyacetamidinyl) dodécane: 26.0

A une solution méthanolique (50 ml) de 3.07 g (22 mmol) de chlorhydrate d'ethyl hydroxyacetimidate, sont ajoutés 2 g (10 mmol) de 1,12-diaminododecane. Le mélange réactionnel est laissé sous agitation à température ambiante durant 24H. La solution est ensuite évaporée sous pression réduite. Le résidu froid est repris avec 100 ml d'eau puis basifié à froid avec NaOH 0.5N. Le précipité formé est essoré puis lavé à l'eau, à l'acétone puis plusieurs fois à l'éther et essoré, pour conduire après séchage à 2.55 g (81%) de 2- hydroxyacetamidinyle 26.0 sous forme de poudre blanche. Point de fusion: 99-100°C.
RMN ¹H (CD₃OD, 250MHz): 1.00 (s, 16H, H₃-H₁₀); 1.27 (m, 4H, H₂ et H₁₁); 2.84 (t, 4H, H₁ et H₁₂); 2.98 (s, 2H, 2 OH); 3.70 (s, 4H, 2H_{2'}).
FT-IR: 1605 (C=N); 3072 (OH); 3290 et 3388 (2NH).
ES⁺ SM: 315 [M+H⁺]

### 1,12-bis[N,N'-(2-iminopyrrolidinyl)]dodécane: 24.0

A un mélange de 2,21 g (26 mmol) de pyrrolidin-2-one et de 0,23 g (10 mmol) de sodium chauffé à 90°C sous azote, sont ajoutés par petites fractions 1,64 g (5 mmol) de 1,12-dibromododécane. Ce mélange réactionnel est ensuite chauffé sous agitation à 120°C durant 4 heures. Après refroidissement à température ambiante, 40 ml d'eau sont additionnés et la solution est extraite avec 40 ml de DCM. La phase organique est ensuite lavée avec une solution aqueuse saturée de NaCl, séchée sur MgSO₄ puis évaporée sous pression réduite. Une purification sur colonne de silice (éluant DCM-méthanol, [18-1]) suivi d'une co-évaporation à température ambiante avec un mélange éther-hexane conduit à 0,655 g de 1,12-bis[*N,N'*-(pyrrolidin-2-one-1-yl)]dodécane, avec un rendement de 39%. A un mélange de 0,336g (lmmol) de ce produit dans 3ml de chloroforme sous azote sont ajoutés goutte à goutte 0,18 ml (2 mmol) de sulfonyl chlorure d'isocyanate dans 3 ml de chloroforme. La réaction est chauffée à 77°C pendant 6 heures. Après addition de 4,5 ml d'eau, la température du mélange réactionnel est ensuite portée à 95°C pendant 4 heures. Après quoi, 5 ml d'eau sont rajoutés et la solution est lavée avec 2 X 5 ml de DCM. La phase aqueuse est ensuite neutralisée par une solution aqueuse de KOH 1N et le solide est filtré, lavé à l'éther puis séché 1heure30 au dessiccateur pour conduire à 0,135 g (40%) de produit.
Point de fusion: 53-55°C
RMN ¹H (DMSOd₆, 250 MHz), δ (ppm) : 1,3 (m, 16H) ; 1,5 (m, 4H) ; 1,8 (q, 4H); 2,3 (t, 4H); 3,2 (t, 4H); 3,3 (t,4H); 8,0 (s, 2H)

### Etude des activités pharmacologiques des composés selon l'invention.

### A. Activité antipaludique in vitro contre P. falciparum et in vivo chez des souris infectées par P. vinckei

On rapporte dans les tableaux 4 et 5 ci-après les résultats des valeurs de CI₅₀ en µM et de DE₅₀ (mg/kg) obtenus avec des composés de l'invention.

**TABLEAU 4**

| **Série A** | | |
|---|---|---|
| **Composé** | **CI₅₀ (microM)** | DE₅₀ (mg/Kg) *P. vinckei* |
| **1.0, 2 HCl** | 0.3 10⁻³ | Nd |
| **1.1** | nd | Nd |
| **1.2** | 0,3 | ip 33 |
| **1.4** | 0,5 | Nd |
| **1.5** | 0,4 | Nd |
| **1.13** | 11 | ip < 20* |
| **1.15** | 3,5 | po = 120 |
| **1.16** | 6,8 | Nd |
| **1.17** | 1,2 | Nd |
| **1.18** | 1,1 | Nd |
| **1.19** | 3,6 | Nd |
| **1.22** | 1,4 | Nd |
| **1.23** | 1,1 | Nd |
| **1.24** | 0,2 | po = 90 |
| **1.27** | 9,2 | Nd |
| **1.28** | 0,2 | Nd |
| **1.30** | 20 10⁻³ | ip =20 |
| **2.0, 2 HCl** | 0,7 10⁻³ | ip = 1,1* po = 40* |
| **3.0** | 4,9 10⁻³ | Nd |
| **4.0, 2 HCl** | 6,3 10⁻³ | Nd |
| **5.0** | 0,1 | Nd |
| **6.0** | 2 10⁻³ | ip = 3,4* po = 62* |
| **6.1** | nd | Nd |
| **6.5** | 12,0 | ip < 20* po ≈ 100* |
| **6.8** | 69,5 | Nd |
| **7.0, 2 HCl** | 43 10⁻³ | Nd |
| **8.0, 2 HCl** | 2,3 10⁻³ | ip = 1,7* |
| **9.0, 2 HCl** | 1 10⁻³ | ip = 7* po = 60* |
| **10.0, 2 HCl** | 1 10⁻³ | ip = 6* po = 95* |
| **11.0, 2 HCl** | 3 10⁻³ | ip = 8* po = 105* |
| **12.0, 2 HBr** | 0,3 10⁻³ | Nd |
| **12.2** | 1,9 | Nd |
| **13.0, 2 HI** | 1,6 10⁻³ | Nd |
| **14.0, 2 HCl** | 21 10⁻³ | Nd |
| **15.0, 2 HBr** | 1,7 10⁻³ | ip = 0,35* po = 45* |
| **16.0, 2 HI** | 0,1 10⁻³ | Nd |
| **17.0, 2 HI** | 0,6 10⁻³ | Nd |

| | | |
|---|---|---|
| * testé après 2 administrations du composé par jour pendant 4 jours | | |

**TABLEAU 5**

| **Série B** | | |
|---|---|---|
| **Composé** | **CI₅₀** (microM) | DE₅₀ (mg/Kg) ***P. vinckei*** |
| **20.0** | 2 10⁻³ | ip = 2,3 * po nd |
| **20.1, 2HCl** | 0,31 | ip = 9,2 po = 90 |
| **20.2** | 4,35 | ip = 10 po = 110 |
| **20.12** | 12 10⁻³ | ip = 4,7 po = 42 |
| **20.14** | 7,1 | ip = 9 po = 62 |
| **21.0** | 3,8 10⁻³ | ip = 2,8* po = 85* |
| **22.0, 2 HCl** | 3 10⁻³ | nd |
| **23.0** | 2,2 10⁻³ | nd |
| **24.0** | 4 10⁻³ | nd |
| **25.0, 2TFA** | 1,9 10⁻³ | nd |
| **26.0** | 9.15 10⁻³ | nd |

| | | |
|---|---|---|
| * testé après 2 administrations du composé par jour pendant 4 jours | | |

La CI₅₀ est la concentration qui inhibe de 50% la croissance *in vitro* de *P. falciparum* (les mesures de CI₅₀ ont été déterminées selon la méthode de Desjardins dans laquelle l'incorporation d'[³H] hypoxanthine dans les acides nucléiques sert d'index de la viabilité cellulaire) (figure 1), la DE₅₀ est la dose efficace pour inhiber de 50% la croissance *in vivo* de *P. vinckei* selon un test suppressif de 4 jours IT correspond à l'index thérapeutique, IT= DL₅₀ (semi chronique)/DE₅₀; ip :administration intrapéritonéale ; po : per os.

Ces résultats montrent que les composés de l'invention possèdent une forte activité antipaludique *in vitro* et *in vivo* ainsi qu'une bonne tolérance et une bonne absorption.

### B. Paramètres pharmacocinétiques chez la souris

On rapporte ci-après les résultats des paramètres pharmacocinétiques après administration par voie intra-péritonéale ou orale chez la souris pour le composé 6.0. Pour la détermination du niveau sérique, on utilise des bio-essais *ex vivo :* brièvement, le médicament est administré à l'animal, puis on procède à des prélèvements sanguins répétés. Les *sera* sont décomplémentés pendant 30 min à 56°C. Le contenu en métabolite actif est alors déterminé par incubation de différentes concentrations (dilution dichotonique) de chaque sérum, en présence de suspensions d'érythrocytes infectés par *P. falciparum,* selon la méthode de DESJARDINS à la [³H] hypoxanthine.

Les résultats sont exprimés en SI₅₀, ce qui correspond au pourcentage de sérum (contenant un métabolite actif) capable d'inhiber la croissance de *P. falciparum* de 50 %.

Cette valeur est alors transformée en concentration sérique, (habituellement exprimée en ng/ml) en testant le composé actif directement (sans passage chez l'animal), sur la même suspension infectée par *P. falciparum* et en déterminant sa valeur de CI₅₀ (en ng/ml) [taux sérique = CI₅₀] (en ng/ml) x 100/SI₅₀ (en %)].

Les résultats sont exprimés en log (taux sérique de médicaments), en fonction du temps, ce qui permet l'évaluation du demi-temps pour la distribution vers le compartiment sérique t_{1/2}(d) ; du demi-temps pour l'élimination du compartiment sérique (t_{½(e)}); de C₀, correspondant au taux sérique extrapolé à l'origine dans la phase d'élimination ; de AUC (qui indique la quantité de drogue circulant dans le courant sanguin) ; et la bio-disponibilité relative dans le mode d'administration par voie orale, versus le mode par voie intrapéritonéale [AUC (po)/AUC (ip)] qui est significatif du degré d'absorption par voie orale.

### Pharmacocinétique de 6.0

On administre à des souris des doses de 17 et 300 mg par kg de 6.0, par voie intrapéritonéale et par voie orale, ce qui correspond à des DL₅₀/3.

Le composé est solubilisé dans du DMSO à 10 %. Les résultats sont donnés dans le tableau 4.

La représentation semi-logarithmique permet de déterminer les paramètres pharmacocinétiques principaux du métabolite actif pour les deux voies d'administration. Les paramètres pharmacocinétiques sont C₀ = 50 ng/ml, t_{1/2} = 16 heures, AUC = 310 ng.h/ml après administation ip à 17 mg/kg, et C₀ = 80 ng/ml, t_{½} = 17h, AUC = 170 ng.h/ml après administration orale à 300 mg/kg.

### Pharmacocinétique de 21.0

Le composé 21.0 a été administré à la souris à des doses de 15 et 100 mg/kg, respectivement par voie intrapéritonéale et par voie orale, DL₅₀/3 en ip et DL₅₀/4 en po.

Après administration intra-péritonéale à 15 mg/kg, on obtient C₀ de 24 ng/ml avec t_{1/2} d'environ 35 heures.

Par voie orale à 100 mg/kg, C₀ est de 16 ng/ml. Le t_{1/2} apparent est de 36 heures.

### C. Activités antibabésioses des composés

Les produits 6.0, 22.0, et 2.0 ont aussi été évalués *in vitro* pour leurs activités contre *Babesia divergens* et *B. canis.* Dans l'un et l'autre cas, les composés 6.0, 22.0, et 2.0 se sont montrés particulèrement actifs (CI₅₀ < 50 nM). Ces résultats indiquent une activité antibabésia puissante pour ce type de composés.

## Revendications

1. Composés présentant une activité anti-parasitaire, notamment antipaludique, **caractérisés en ce qu**'ils répondent à la formule générale (I) 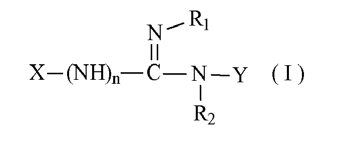 dans laquelle
X = R₄ et Y représente un groupe de formule (III) 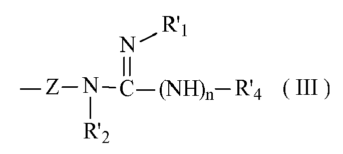 avec :
. Z est un groupe -(CH2)ₘ-avec m = 8 à 21,
n = 0 ou 1,
. R₂ et R'₂, identiques ou différents l'un de l'autre, étant choisis parmi H, alkyl, CO-O-CH₂-aryl, CO-O-alkyl cycloalkyl,et
. R₁ et R₄ et/ou R'₁ et R'₄ forment ensemble un groupe -(CH₂)ₚ, p étant un entier de 1 à 5, un ou plusieurs atomes d'hydrogène pouvant être remplacés par un alkyl inférieur, et R₂ et R'₂ représentant H, ou bien R₄ et R₂ et/ou R'₄ et R'₂ forment ensemble un groupe -(CH₂)ₚ, un ou plusieurs H pouvant être remplacés par un alkyl inférieur, R₁ et R'₁ représentant H, et les sels pharmacologiquement acceptables de ces composés.

2. Composés selon la revendication 1, **caractérisés en ce qu**'ils répondent à la formule (IX) 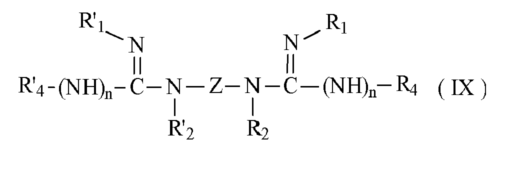 dans laquelle n, Z, R₁, R'₁, R₂, R'₂, R₄ et R'₄ sont tels que définis dans la revendication 1.

3. Composés selon la revendication 2, **caractérisés en ce que** R₁ et R₄ et/ou R'₁ et R'₄ représentent ensemble un groupe -(CH₂)ₚ- où p est un entier de 3 à 5 et R₂ et R'₂ représentent H.

4. Composé selon la revendication 2 ou 3, **caractérisé en ce qu**'il répond à la formule (XI)

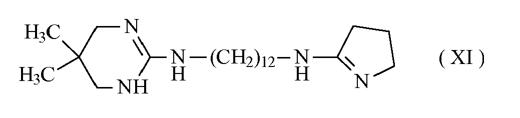

5. Compositions pharmaceutiques, **caractérisées en ce qu**'elles renferment une quantité efficace d'au moins un composé tel que défini dans l'une quelconque des revendications 1 à 4 en association avec un véhicule pharmaceutique inerte.

6. Compositions pharmaceutiques selon la revendication précédente, **caractérisées en ce qu**'elles sont administrables par voie orale, par voie injectable, ou encore par voie rectale.

7. Compositions selon la revendication 5 ou 6 pour le traitement des maladies infectieuses, en particulier du paludisme, **caractérisées en ce qu**'elles comprennent une quantité efficace des composés selon l'une quelconque des revendications 1 à 4.

8. Utilisation d'au moins un composé selon l'une quelconque des revendications 1 à 4 pour fabriquer des médicaments pour le traitement des maladies anti-parasitaires, en particulier du paludisme.

9. Utilisation d'au moins un composé selon l'une quelconque des revendications 1 à 4 pour fabriquer des médicaments pour le traitement des maladies anti-parasitaires, en particulier du paludisme et des babésioses.
